# EUROPÄISCHE PATENTSCHRIFT

(11) **EP 0 891 336 B1**
(45) Veröffentlichungstag und Bekanntmachung des Hinweises auf die Patenterteilung: **05.06.2002**
(21) Anmeldenummer: 97907086.9
(22) Anmeldetag: 10.03.1997
(51) Int. Cl.: C07D 239/54, C07C 271/22, C07C 275/24, A01N 43/54

(54) **SUBSTITUIERTE 1-METHYL-3-BENZYLURACILE**
SUBSTITUTED 1-METHYL-3-BENZYLURACILS
1-METHYLE-3-BENZYLURACILES SUBSTITUES

(30) Priorität: 27.03.1996 DE 19612032
(43) Veröffentlichungstag der Anmeldung: 20.01.1999
(73) Patentinhaber: BASF AKTIENGESELLSCHAFT, 67056 Ludwigshafen (DE)
(72) Erfinder: MENKE, Olaf, D-67317 Altleiningen (DE); HAMPRECHT, Gerhard, D-69469 Weinheim (DE); HEISTRACHER, Elisabeth, D-68159 Mannheim (DE); KLINTZ, Ralf, D-44801 Bochum (DE); SCHÄFER, Peter, D-67308 Ottersheim (DE); ZAGAR, Cyrill, D-67061 Ludwigshafen (DE); MENGES, Markus, D-68161 Mannheim (DE); WESTPHALEN, Karl-Otto, D-67346 Speyer (DE); WALTER, Helmut, D-67283 Obrigheim (DE); MISSLITZ, Ulf, D-67433 Neustadt (DE)
(86) Internationale Anmeldenummer: EP9701203
(87) Internationale Veröffentlichungsnummer: WO9735845

(56) Entgegenhaltungen:
- WO-A-95/04461

## Beschreibung

Die vorliegende Erfindung betrifft neue substituierte 1-Methyl-3-benzyluracile der Formel I 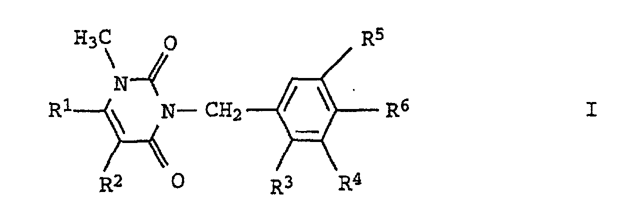 in der die Variablen folgende Bedeutungen haben:
- R¹: C₁-C₄-Halogenalkyl;
- R²: Wasserstoff;
- R³: Halogen;
- R⁴: Cyano, Halogen oder C₁-C₄-Alkoxy;
- R⁵: Wasserstoff;
- R⁶: C₁-C₆-Alkoxy oder C₃-C₆-Alkenyloxy, wobei jeder dieser beiden Reste einen oder zwei der folgenden Substituenten tragen kann:
- die Phenylgruppe, die unsubstituiert sein oder ein bis drei Substituenten tragen kann, jeweils ausgewählt aus der Gruppe bestehend aus Halogen, Nitro, Cyano, C₁-C₆-Alkyl, C₁-C₆-Halogenalkyl, C₁-C₆-Alkoxy und (C₁-C₆-Alkoxy)carbonyl,
- eine Gruppe -CO-R⁹, -CO-OR⁹, -CO-SR⁹ oder -CO-N(R⁹)R¹⁰,
- eine Gruppe =N-OR²⁰ oder -C(R²¹)=N-OR²⁰;
- CH(NR²⁷), -CH=C(R¹⁴)-CO-R¹⁵, -CH=C(R¹⁴)-CO-N(R¹⁹)-OR²⁰, -CH=C(R¹⁴)-C(R²¹)=N-OR²⁰, -N(R²³)R²⁴, -CO-N(R¹⁹)-OR²⁰, -C(R²¹)=N-OR²⁰, -COO-R²² oder -CON(R²³)R²⁴;
- R⁹: C₁-C₃-Alkyl, C₃-C₄-Alkenyl oder (C₁-C₃-Alkoxy)carbonyl-C₁-C₃-alkyl;
- R¹⁰: Wasserstoff, C₁-C₃-Alkoxy, (C₁-C₃-Alkoxy)carbonyl-C₁-C₃-alkoxy oder C₃-C₄-Alkenyloxy;
- R¹⁴: Wasserstoff, Halogen oder C₁-C₆-Alkyl;
- R¹⁵: O-R²² oder -N(R²³)R²⁴;
- R¹⁹: Wasserstoff, C₁-C₃-Alkyl oder C₃-C₄-Alkenyl, wobei die beiden letzten Gruppen jeweils einen der folgenden Reste tragen können: Chlor, (C₁-C₆-Alkyl)carbonyl, (C₁-C₆-Alkoxy)carbonyl oder (C₃-C₆-Alkenyloxy) carbonyl;
oder (C₁-C₆-Alkylamino)carbonyl oder Di-(C₁-C₆-alkyl)aminocarbonyl;
- R²⁰: C₁-C₄-Alkyl, C₃-C₄-Alkenyl oder (C₁-C₃-Alkoxy)carbonyl-C₁-C₃-alkyl;
- R²¹: C₃-C₄-Alkinyloxy, C₁-C₃-Alkoxy oder C₃-C₆-Alkenyloxy, wobei die beiden letzten Reste jeweils einen der folgenden Substituenten tragen können: (C₁-C₃-Alkoxy)carbonyl, (C₁-C₃-Alkylamino)carbonyl oder Dimethylaminocarbonyl;
- R²²: eine der Bedeutungen von R¹⁹;
- R²³,: R²⁴ unabhängig voneinander Wasserstoff, C₁-C₃-Alkyl, (C₁-C₃-Alkyl)carbonyl, C₁-C₃-Alkylsulfonyl oder Phenylsulfonyl, dessen Phenylring einen Halogen-, C₁-C₃-Alkyl- oder C₁-C₃-Alkoxy-Substituenten trägt;
- R²⁷: C₁-C₆-Alkoxy, C₃-C₆-Alkenyloxy, (C₁-C₆-Alkoxy)carbonyl-C₁-C₆-alkoxy oder Benzyloxy;
sowie die landwirtschaftlich brauchbaren Salze und Enolether der Verbindungen I.

Außerdem betrifft die Erfindung
- die Verwendung der Verbindungen I als Herbizide und/oder zur Desikkation und/oder Defoliation von Pflanzen,
- herbizide Mittel und Mittel zur Desikkation und/oder Defoliation von Pflanzen, welche die Verbindungen I als wirksame Substanzen enthalten,
- Verfahren zur Herstellung der Verbindungen I und von herbiziden Mitteln und Mitteln zur Desikkation und/oder Defoliation von Pflanzen unter Verwendung der Verbindungen I,
- Verfahren zur Bekämpfung von unerwünschtem Pflanzenwuchs und zur Desikkation und/oder Defoliation von Pflanzen mit den Verbindungen I, sowie
- neue Zwischenprodukte der Formeln III und IV.

In der WO 95/04461 werden bereits 3-Benzyl-1-methyl-6-trifluormethyluracile der Formel II 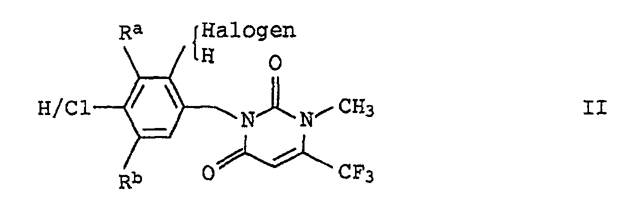 wobei
- R^{a}: für Wasserstoff, Cyano, Halogen, C₁-C₃-Alkoxy, C₁-C₃-Alkylaminocarbonyl oder Propargyloxy und
- R^{b}: für Wasserstoff, Cyano, Halogen, C₁-C₃-Alkylaminocarbonyl oder Carboxy stehen,
als Herbizide beschrieben.

Die herbizide Wirkung der bekannten Verbindungen bezüglich der Schadpflanzen ist jedoch nicht immer voll befriedigend. Aufgabe der vorliegenden Erfindung war es demnach, neue herbizid wirksame Verbindungen bereitzustellen, mit denen sich unerwünschte Pflanzen besser als bisher gezielt bekämpfen lassen. Die Aufgabe erstreckt sich auch auf die Bereitstellung neuer desikkant/defoliant wirksamer Verbindungen.

Demgemäß wurden die eingangs definierten substituierten 1-Methyl-3-benzyluracile der Formel I gefunden. Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des weiteren wurde gefunden, daß die Verbindungen I auch zur Defoliation und Desikkation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und/oder Defoliation von Pflanzen, Verfahren zur Herstellung dieser Mittel und Verfahren zur Desikkation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die Verbindungen I können auch in den tautomeren Formeln I' und I" [-N(CH₃)-CO-↔ -N=C(OCH₃)-] als Enolether geschrieben werden:

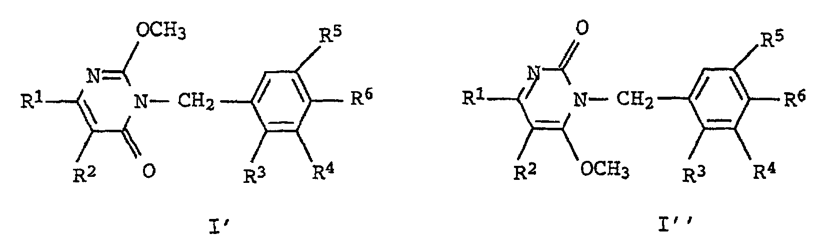

Unter landwirtschaftlich brauchbaren Salzen kommen vor allem die Salze derjenigen Kationen oder die Säureadditionssalze derjenigen Säuren in Betracht, deren Kationen beziehungsweise Anionen die herbizide Wirkung der Verbindungen I nicht negativ beeinträchtigen. So kommen als Kationen insbesondere die Ionen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Phosphat, Nitrat, Hydrogencarbonat, Carbonat, Hexafluorosilikat, Hexafluorophosphat, Benzoat, sowie die Anionen von C₁-C₄-Alkansäuren, vorzugsweise Formiat, Acetat, Propionat und Butyrat.

Die bei der Definition von R¹, R⁴, R⁶, R⁹, R¹⁰, R¹⁴, R¹⁹ bis R²⁴ und R²⁷ genannten organischen Molekülteile stellen Sammelbegriffe für individuelle Aufzählungen der einzelnen Gruppenmitglieder dar. Sämtliche Kohlenstoffketten, also alle Alkyl-, Halogenalkyl-, Alkoxy-, Alkylsulfonyl-, Alkylamino-, Dialkylamino-, Alkenyl-, Alkenyloxy- und Alkinyloxy-Teile können geradkettig oder verzweigt sein.

Halogenierte Substituenten tragen vorzugsweise ein bis fünf gleiche oder verschiedene Halogenatome.

Die Bedeutung Halogen steht jeweils für Fluor, Brom, Chlor oder Iod, insbesondere für Fluor oder Chlor.

Ferner stehen beispielsweise:
- C₁-C₄-Alkyl für: Methyl, Ethyl, n-Propyl, 1-Methylethyl, n-Butyl, 1-Methylpropyl, 2-Methylpropyl oder 1,1-Dimethylethyl, insbesondere für Methyl oder Ethyl;
- C₁-C₄-Halogenalkyl für: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl, insbesondere für Chlormethyl, Fluormethyl, Difluormethyl, Trifluormethyl, 2-Fluorethyl, 2-Chlorethyl oder 2,2,2-Trifluorethyl;
- C₁-C₆-Alkyl für: C₁-C₄-Alkyl wie vorstehend genannt, sowie z.B. n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, insbesondere für Methyl, Ethyl, n-Propyl, 1-Methylethyl, n-Butyl, 1,1-Dimethylethyl, n-Pentyl oder n-Hexyl;
- C₁-C₆-Halogenalkyl für: C₁-C₆-Alkyl wie vorstehend genannt, das partiell oder vollständig durch Fluor, Chlor, Brom und/oder Iod substituiert ist, also z.B. einen der unter C₁-C₄-Halogenalkyl genannten Reste oder für 5-Fluor-1-pentyl, 5-Chlor-1-pentyl, 5-Brom-1-pentyl, 5-Iod-1-pentyl, 5,5,5-Trichlor-1-pentyl, Undecafluorpentyl, 6-Fluor-1-hexyl, 6-Chlor-1-hexyl, 6-Brom-1-hexyl, 6-Iod-1-hexyl, 6,6,6-Trichlor-1-hexyl oder Dodecafluorhexyl, insbesondere für Chlormethyl, Fluormethyl, Difluormethyl, Trifluormethyl, 2-Fluorethyl, 2-Chlorethyl oder 2,2,2-Trifluorethyl;
- (C₁-C₆-Alkyl)carbonyl für: Methylcarbonyl, Ethylcarbonyl, n-Propylcarbonyl, 1-Methylethylcarbonyl, n-Butylcarbonyl, 1-Methylpropylcarbonyl, 2-Methylpropylcarbonyl, 1,1-Dimethylethylcarbonyl, n-Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, n-Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl, insbesondere für Methylcarbonyl, Ethylcarbonyl oder 1-Methylethylcarbonyl;
- C₁-C₄-Alkoxy für: OCH₃, OC₂H₅, n-Propoxy, OCH(CH₃)₂, n-Butoxy, 1-Methylpropoxy, OCH₂-CH(CH₃)₂ oder OC(CH₃)₃, insbesondere für OCH₃ oder OC₂H₅;
- C₁-C₆-Alkoxy für: C₁-C₄-Alkoxy wie vorstehend genannt, sowie z.B. n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy, insbesondere für Methoxy, Ethoxy oder 1-Methylethoxy;
- (C₁-C₆-Alkoxy)carbonyl für: (C₁-C₄-Alkoxy)carbonyl wie vorstehend genannt, sowie z.B. n-Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, n-Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl, insbesondere für Methoxycarbonyl, Ethoxycarbonyl oder 1-Methylethoxycarbonyl;
- C₁-C₃-Alkylsulfonyl für: Methylsulfonyl, Ethylsulfonyl, n-Propylsulfonyl oder 1-Methylethylsulfonyl, insbesondere für Methylsulfonyl;
- (C₁-C₃-Alkylamino)carbonyl für: CO-NH-CH₃, CO-NH-C₂H₅, n-Propylaminocarbonyl oder CO-NH-CH(CH₃)₂, insbesondere für CO-NH-CH₃ oder CO-NH-C₂H₅;
- (C₁-C₆-Alkylamino)carbonyl für: (C₁-C₃-Alkylamino)carbonyl wie vorstehend genannt sowie z.B. n-Butylaminocarbonyl, 1-Methylpropylaminocarbonyl, CO-NH-CH₂-CH(CH₃)₂, CO-NH-C(CH₃)₃, n-Pentylaminocarbonyl, 1-Methylbutylaminocarbonyl, 2-Methylbutylaminocarbonyl, 3-Methylbutylaminocarbonyl, 2,2-Dimethylpropylaminocarbonyl, 1-Ethylpropylaminocarbonyl, n-Hexylaminocarbonyl, 1,1-Dimethylpropylaminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylaminocarbonyl, 1,1-Dimethylbutylaminocarbonyl, 1,2-Dimethylbutylaminocarbonyl, 1,3-Dimethylbutylaminocarbonyl,. 2,2-Dimethylbutylaminocarbonyl, 2,3-Dimethylbutylaminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethylpropylaminocarbonyl, 1-Ethyl-1-methylpropylaminocarbonyl oder 1-Ethyl-2-methylpropylaminocarbonyl, insbesondere für CO-NH-CH₃, CO-NH-C₂H₅ oder CO-NH-CH(CH₃)₂;
- Di-(C₁-C₆-alkyl)aminocarbonyl für: z.B. N,N-Dimethylaminocarbonyl, N,N-Diethylaminocarbonyl, N,N-Dipropylaminocarbonyl, N,N-Di-(1-methylethyl)aminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)-aminocarbonyl, N-Butyl-N-methylaminocarbonyl, N-Methyl-N-(1-methylpropyl)aminocarbonyl, N-Methyl-N-(2-methylpropyl)-aminocarbonyl, N-(1,1-Dimethylethyl)-N-methylaminocarbonyl, N-Ethyl-N-propylaminocarbonyl, N-Ethyl-N-(1-methylethyl)-aminocarbonyl, N-Butyl-N-ethylaminocarbonyl, N-Ethyl-N-(1-methylpropyl)aminocarbonyl, N-Ethyl-N-(2-methylpropyl)-aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)aminocarbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)aminocarbonyl, N-Butyl-N-(2-methylpropyl)aminocarbonyl, N-Butyl-N-(1,1-dimethylethyl)aminocarbonyl, N-(1-Methylpropyl)-N-(2-methylpropyl)aminocarbonyl, N-(1,1-Dimethylethyl)-N-(1-methylpropyl)aminocarbonyl oder N-(1,1-Dimethylethyl)-N-(2-methylpropyl)aminocarbonyl, insbesondere für N,N-Dimethylaminocarbonyl oder N,N-Diethylaminocarbonyl;
- (C₁-C₆-Alkoxy)carbonyl-C₁-C₆-alkoxy für: durch (C₁-C₆-Alkoxy)-carbonyl wie vorstehend genannt substituiertes C₁-C₆-Alkoxy, also z.B. für OCH₂-CO-OCH₃, OCH₂-CO-OC₂H₅, OCH₂-CO-OCH₂-C₂H₅, OCH₂-CO-OCH(CH₃)₂, n-Butoxycarbonyl-methoxy, 1-(Methoxycarbonyl)ethoxy, 2-(Methoxycarbonyl)ethoxy, 2-(Ethoxycarbonyl)ethoxy, 2-(n-Propoxycarbonyl)ethoxy, 2-(n-Butoxycarbonyl)ethoxy, 3-(Methoxycarbonyl)propoxy, 3-(Ethoxycarbonyl)propoxy, 3-(n-Propoxycarbonyl)propoxy, 3-(n-Butoxycarbonyl)propoxy, 4-(Methoxycarbonyl)butoxy, 4-(Ethoxycarbonyl)butoxy, 4-(n-Propoxycarbonyl)butoxy, 4-(n-Butoxycarbonyl)butoxy, 5-(Methoxycarbonyl)pentoxy, 5-(Ethoxycarbonyl)pentoxy, 5-(n-Propoxycarbonyl)pentoxy, 5-(n-Butoxycarbonyl)butoxy, 6-(Methoxycarbonyl)hexoxy, 6-(Ethoxycarbonyl)hexoxy, 6-(n-Propoxycarbonyl)hexoxy oder 6-(n-Butoxycarbonyl)hexoxy, insbesondere für OCH₂-CO-OCH₃ oder 1-(Methoxycarbonyl)ethoxy;
- (C₁-C₃-Alkoxy)carbonyl-C₁-C₃-alkyl für: durch (C₁-C₃-Alkoxy)-carbonyl substituiertes C₁-C₃-Alkyl, also z.B. für Methoxycarbonylmethyl, Ethoxycarbonylmethyl, 1-(Methoxycarbonyl)ethyl, 2-(Methoxycarbonyl)ethyl, 2-(Ethoxycarbonyl)ethyl oder 3-(Methoxycarbonyl)propyl;
- C₃-C₄-Alkenyl für: z.B. Prop-2-en-1-yl, n-Buten-4-yl, 1-Methyl-prop-2-en-1-yl, 2-Methyl-prop-2-en-1-yl oder 2-Buten-1-yl, insbesondere für Prop-2-en-1-yl oder n-Buten-4-yl;
- C₃-C₆-Alkenyloxy für: Prop-1-en-1-yloxy, Prop-2-en-1-yloxy, 1-Methylethenyloxy, n-Buten-1-yloxy, n-Buten-2-yloxy, n-Buten-3-yloxy, 1-Methyl-prop-1-en-1-yloxy, 2-Methylprop-1-en-1-yloxy, 1-Methyl-prop-2-en-1-yloxy, 2-Methylprop-2-en-1-yloxy, n-Penten-1-yloxy, n-Penten-2-yloxy, n-Penten-3-yloxy, n-Penten-4-yloxy, 1-Metyl--but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methyl-but-3-en-1-yloxy, 2-Methyl-but-3-en-1-yloxy, 3-Methyl-but-3-en-1-yloxy, 1,1-Dimethyl-prop-2-en-1-yloxy, 1,2-Dimethyl-prop-1-en-1-yloxy, 1,2-Dimethyl-prop-2-en-1-yloxy, 1-Ethyl-prop-1-en-2-yloxy, 1-Ethyl-prop-2-en-1-yloxy, n-Hex-1-en-1-yloxy, n-Hex-2-en-1-yloxy, n-Hex-3-en-1-yloxy, n-Hex-4-en-1-yloxy, n-Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloxy, 1-Methyl-pent-2-en-1-yloxy, 2-Methyl-pent-2-en-1-yloxy, 3-Methyl-pent-2-en-1-yloxy, 4-Methyl-pent-2-en-1-yloxy, 1-Methyl-pent-3-en-1-yloxy, 2-Methyl-pent-3-en-1-yloxy, 3-Methyl-pent-3-en-1-yloxy, 4-Methyl-pent-3-en-1-yloxy, 1-Methyl-pent-4-en-1-yloxy, 2-Methyl-pent-4-en-1-yloxy, 3-Methyl-pent-4-en-1-yloxy, 4-Methyl-pent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethylbut-3-en-1-yloxy, 2-Ethyl-but-1-en-1-yloxy, 2-Ethyl-but-2-en-1-yloxy, 2-Ethyl-but-3-en-1-yloxy, 1,1,2-Trimethyl-prop-2-en-1-yloxy, 1-Ethyl-1-methyl-prop-2-en-1-yloxy, 1-Ethyl-2-methyl-prop-1-en-1-yloxy oder 1-Ethyl-2-methyl-prop-2-en-1-yloxy, insbesondere für Prop-2-en-1-yloxy;
- C₃-C₄-Alkinyloxy für: Prop-1-in-1-yloxy, Prop-2-in-1-yloxy, n-But-1-in-1-yloxy, n-But-1-in-3-yloxy, n-But-1-in-4-yloxy oder n-But-2-in-1-yloxy, insbesondere für Prop-2-in-1-yloxy;
- (C₃-C₆-Alkenyl)oxycarbonyl für: Prop-1-en-1-yloxycarbonyl, Prop-2-en-1-yloxycarbonyl, 1-Methylethenyloxycarbonyl, n-Buten-1-yloxycarbonyl, n-Buten-2-yloxycarbonyl, n-Buten-3-yloxycarbonyl, 1-Methyl-prop-1-en-1-yloxycarbonyl, 2-Methylprop-1-en-1-yloxycarbonyl, 1-Methyl-prop-2-en-1-yloxycarbonyl, 2-Methyl-prop-2-en-1-yloxycarbonyl, n-Penten-1-yloxycarbonyl, n-Penten-2-yloxycarbonyl, n-Penten-3-yloxycarbonyl, n-Penten-4-yloxycarbonyl, 1-Methyl-but-1-en-1-yloxycarbonyl, 2-Methyl-but-1-en-1-yloxycarbonyl, 3-Methylbut-1-en-1-yloxycarbonyl, 1-Methyl-but-2-en-1-yloxycarbonyl, 2-Methyl-but-2-en-1-yloxycarbonyl, 3-Methyl-but-2-an-1-yloxycarbonyl, 1-Methyl-but-3-en-1-yloxycarbonyl, 2-Methyl-but-3-en-1-yloxycarbonyl, 3-Methyl-but-3-en-1-yloxycarbonyl, 1,1-Dimethyl-prop-2-en-1-yloxycarbonyl, 1,2-Dimethyl-prop-1-en-1-yloxycarbonyl, 1,2-Dimethyl-prop-2-en-1-yloxycarbonyl, 1-Ethyl-prop-1-en-2-yloxycarbonyl, 1-Ethyl-prop-2-en-1-yloxycarbonyl, n-Hex-1-en-1-yloxycarbonyl, n-Hex-2-en-1-yloxycarbonyl, n-Hex-3-en-1-yloxycarbonyl, n-Hex-4-en-1-yloxycarbonyl, n-Hex-5-en-1-yloxycarbonyl, 1-Methyl-pent-1-en-1-yloxycarbonyl, 2-Methyl-pent-1-en-1-yloxycarbonyl, 3-Methylpent-1-en-1-yloxycarbonyl, 4-Methyl-pent-1-en-1-yloxycarbonyl, 1-Methyl-pent-2-en-1-yloxycarbonyl, 2-Methyl-pent-2-en-1-yloxycarbonyl, 3-Methyl-pent-2-en-1-yloxycarbonyl, 4-Methyl-pent-2-en-1-yloxycarbonyl, 1-Methyl-pent-3-en-1-yloxycarbonyl, 2-Methyl-pent-3-en-1-yloxycarbonyl, 3-Methylpent-3-en-1-yloxycarbonyl, 4-Methyl-pent-3-en-1-yloxycarbonyl, 1-Methyl-pent-4-en-1-yloxycarbonyl, 2-Methyl-pent-4-en-1-yloxycarbonyl, 3-Methyl-pent-4-en-1-yloxycarbonyl, 4-Methyl-pent-4-en-1-yloxycarbonyl, 1,1-Dimethyl-but-2-en-1-yloxycarbonyl, 1,1-Dimethyl-but-3-en-1-yloxycarbonyl, 1,2-Dimethyl-but-1-en-1-yloxycarbonyl, 1,2-Dimethyl-but-2-en-1-yloxycarbonyl, 1,2-Dimethyl-but-3-en-1-yloxycarbonyl, 1,3-Dimethyl-but-1-en-1-yloxycarbonyl, 1,3-Dimethyl-but-2-en-1-yloxycarbonyl, 1,3-Dimethyl-but-3-en-1-yloxycarbonyl, 2,2-Dimethyl-but-3-en-1-yloxycarbonyl, 2,3-Dimethyl-but-1-en-1-yloxycarbonyl, 2,3-Dimethyl-but-2-en-1-yloxycarbonyl, 2,3-Dimethyl-but-3-en-1-yloxycarbonyl, 3,3-Dimethyl-but-1-en-1-yloxycarbonyl, 3,3-Dimethyl-but-2-en-1-yloxycarbonyl, 1-Ethyl-but-1-en-1-yloxycarbonyl, 1-Ethyl-but-2-en-1-yloxycarbonyl, 1-Ethyl-but-3-en-1-yloxycarbonyl, 2-Ethyl-but-1-en-1-yloxycarbonyl, 2-Ethyl-but-2-en-1-yloxycarbonyl, 2-Ethyl-but-3-en-1-yloxycarbonyl, 1,1,2-Trimethyl-prop-2-en-1-yloxycarbonyl, 1-Ethyl-1-methyl-prop-2-en-1-yloxycarbonyl, 1-Ethyl-2-methyl-prop-1-en-1--yloxycarbonyl oder 1-Ethyl-2-methyl-prop-2-en-1-yloxycarbonyl, insbesondere für Prop-2-en-1-yloxycarbonyl.

Im Hinblick auf die Verwendung der erfindungsgemäßen Verbindungen der Formel I als Herbizide und/oder als defoliant/desikkant wirksame Verbindungen haben die Variablen vorzugsweise folgende Bedeutungen, und zwar jeweils für sich allein oder in Kombination:
- R¹: Trifluormethyl;
- R³: Chlor;
- R⁴: Chlor, Cyano oder C₁-C₃-Alkoxy; besonders bevorzugt ist Chlor oder Cyano, ganz besonders Chlor;
- R⁶: C₁-C₆-Alkoxy, das einen oder zwei der folgenden Substituenten trägt:
- eine Gruppe -CO-OR⁹, -CO-N(R⁹)R¹⁰, =NOR²⁰ oder -C(R²¹)=N-OR²⁰;
   oder C₃-C₆-Alkenyloxy, -CH(NR²⁷), -CH=C(R¹⁴)-CO-R¹⁵, -CH=C(R¹⁴)-CO-N(R¹⁹)-OR²⁰, -CH=C(R¹⁴)-C(R²¹)=N-OR²⁰, -N(R²³)R²⁴, -CO-N(R¹⁹)-OR²⁰ oder -C(R²¹)=NOR²⁰;
- R⁷: für Wasserstoff, C₁-C₆-Alkyl, C₃-C₆-Alkenyl, (C₁-C₆-Alkoxy)-carbonyl-C₁-C₆-alkyl oder Phenyl, das unsubstituiert sein oder einen bis drei Reste tragen kann, jeweils ausgewählt aus der Gruppe bestehend aus Halogen, Nitro, C₁-C₆-Alkyl, C₁-C₆-Alkoxy und (C₁-C₆-Alkyl)carbonyl,
insbesondere C₁-C₃-Alkyl, C₃-C₄-Alkenyl oder (C₁-C₃-Alkoxy) carbonyl-C₁-C₃-alkyl;
- R⁸: für Wasserstoff, C₁-C₃-Alkyl, C₁-C₃-Alkoxy, (C₁-C₃-Alkoxy)-carbonyl-C₁-C₃-alkoxy oder C₃-C₄-Alkenyloxy,
insbesondere Wasserstoff, C₁-C₃-Alkoxy, (C₁-C₃-Alkoxy)-carbonyl-C₁-C₃-alkoxy oder C₃-C₄-Alkenyloxy;
- R¹⁴: Wasserstoff, Chlor oder Methyl;
besonders bevorzugt ist Chlor;
- R¹⁹: Wasserstoff, C₁-C₃-Alkyl oder C₃-C₄-Alkenyl, wobei die beiden letzten Gruppen jeweils einen der folgenden Reste tragen können: Chlor, (C₁-C₃-Alkoxy)carbonyl oder (C₃-C₄-Alkenyloxy) carbonyl;
oder (C₁-C₂-Alkyl)aminocarbonyl oder Dimethylaminocarbonyl;
- R²²: Wasserstoff, C₁-C₃-Alkyl oder C₃-C₄-Alkenyl, wobei die beiden letzten Gruppen jeweils einen der folgenden Reste tragen können: Chlor, (C₁-C₃-Alkoxy)carbonyl oder (C₃-C₄-Alkenyloxy)carbonyl;
oder (C₁-C₂-Alkyl)aminocarbonyl oder Dimethylaminocarbonyl;
- R²³, R²⁴: unabhängig voneinander Wasserstoff, Methyl, Ethyl, Methylsulfonyl oder Ethylsulfonyl;
- R²⁷: C₁-C₃-Alkoxy, C₃-C₄-Alkenyloxy, (C₁-C₃-Alkoxy)carbonyl-C₁-C₃-alkoxy oder Benzyloxy.

Bevorzugt sind ferner
- diejenigen Verbindungen I, bei denen R⁶ C₁-C₄-Alkoxy, das eine oder zwei Gruppen -CO-OR⁹, -CO-N(R⁹)R¹⁰, =N-OR²⁰ und/oder -C(R²¹)=N-OR²⁰ trägt, bedeutet, und
- diejenigen Verbindungen I, bei denen R⁶ -CH(NR²⁷) bedeutet, und
- diejenigen Verbindungen I, bei denen R⁶ -CH=C(R¹⁴)-CO-R¹⁵ bedeutet, und
- diejenigen Verbindungen I, bei denen R⁶ -CH=C(R¹⁴)-CO-N(R¹⁹)-OR²⁰ bedeutet, und
- diejenigen Verbindungen I, bei denen R⁶ -CH=C(R¹⁴)-C(R²¹)=N-OR²⁰ bedeutet.

Ganz besonders bevorzugt sind die in der folgenden Tabelle 1 aufgeführten Verbindungen Ia (≙ I mit R¹ = Trifluormethyl, R³, R⁴ = Chlor):

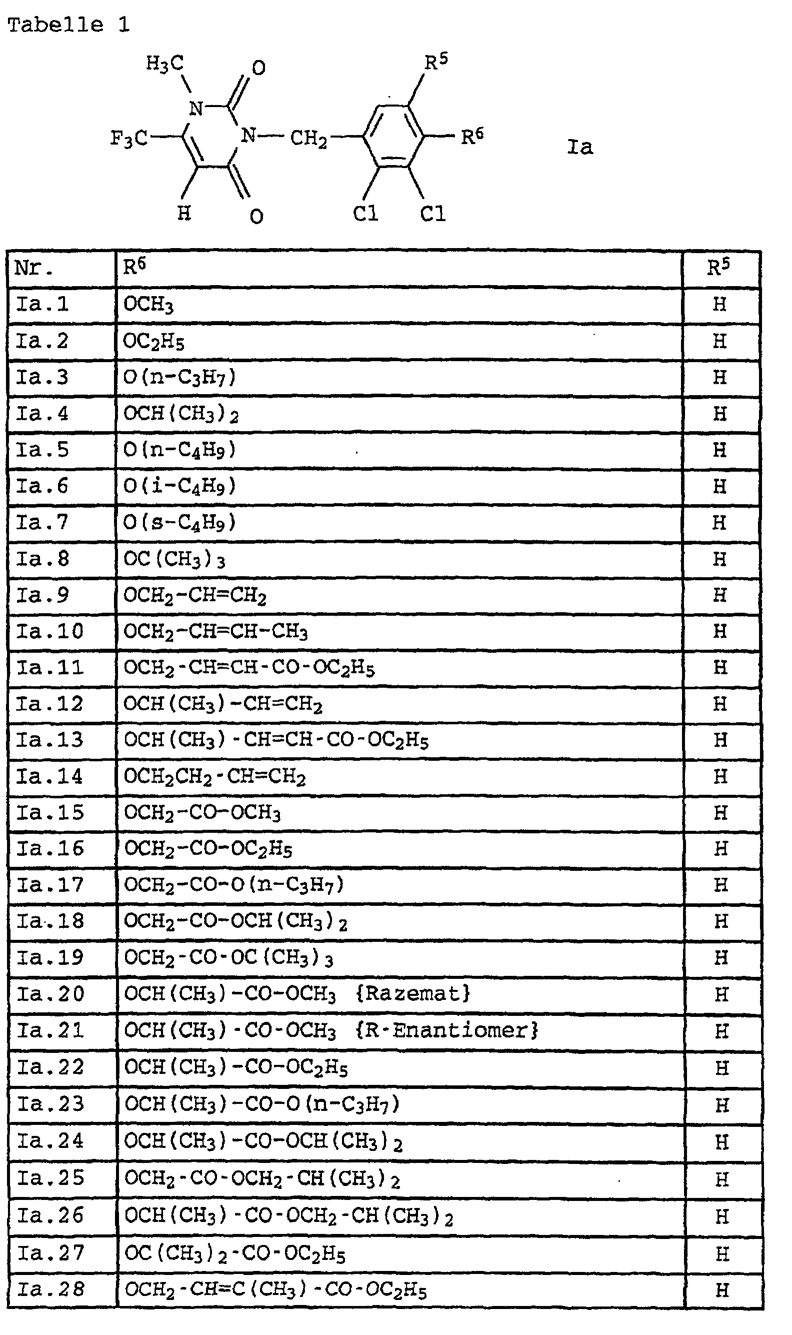

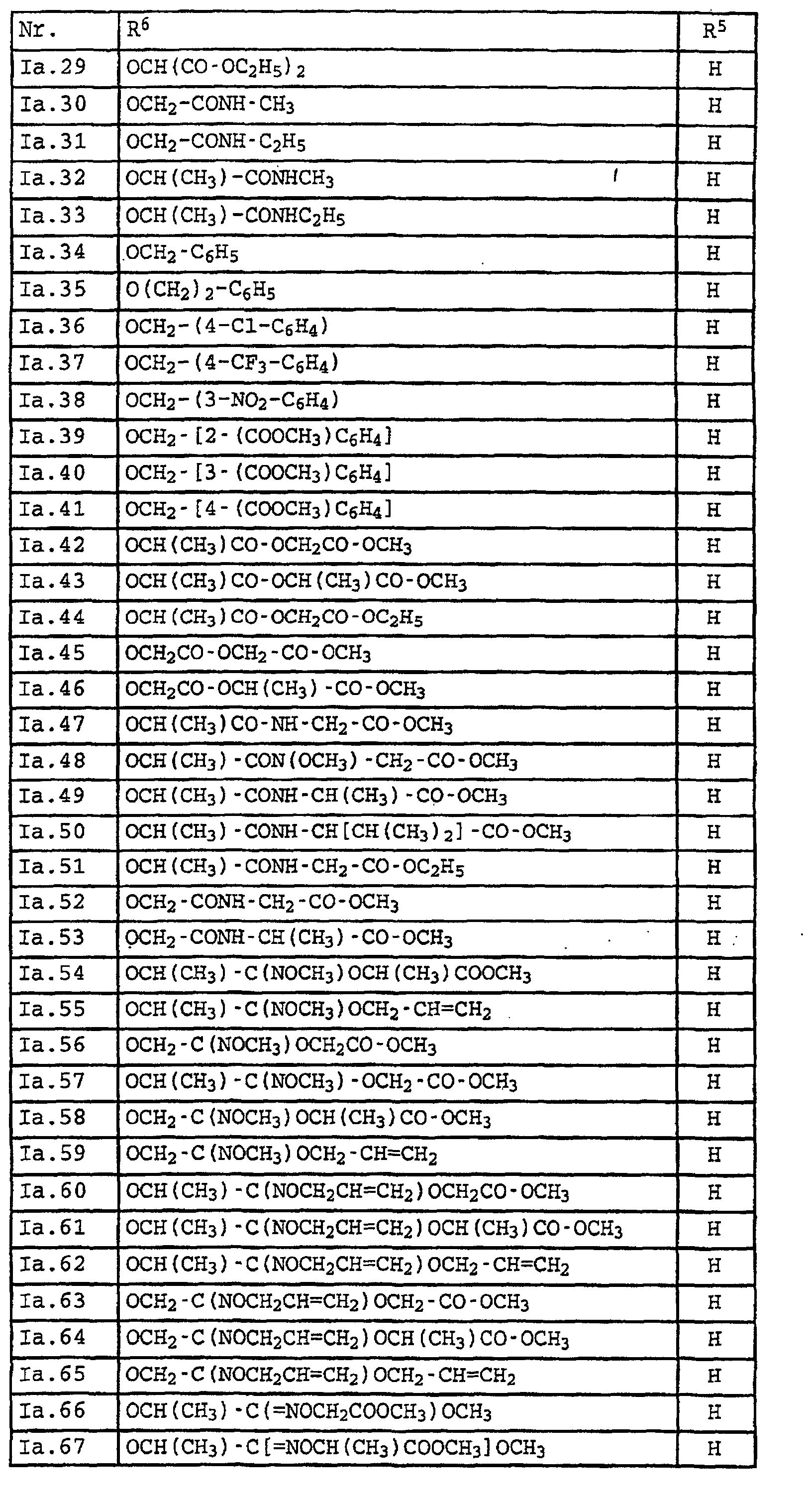

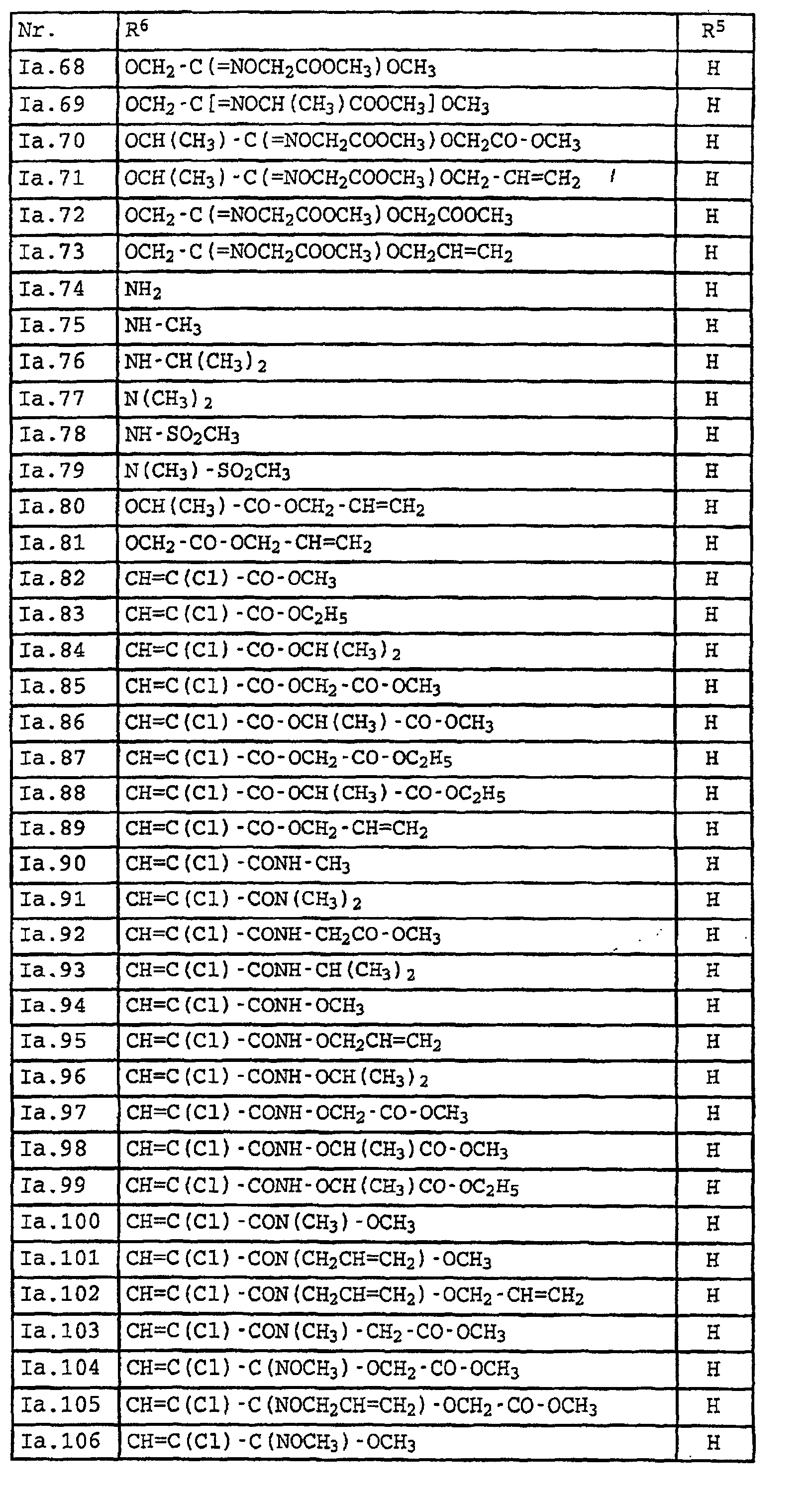

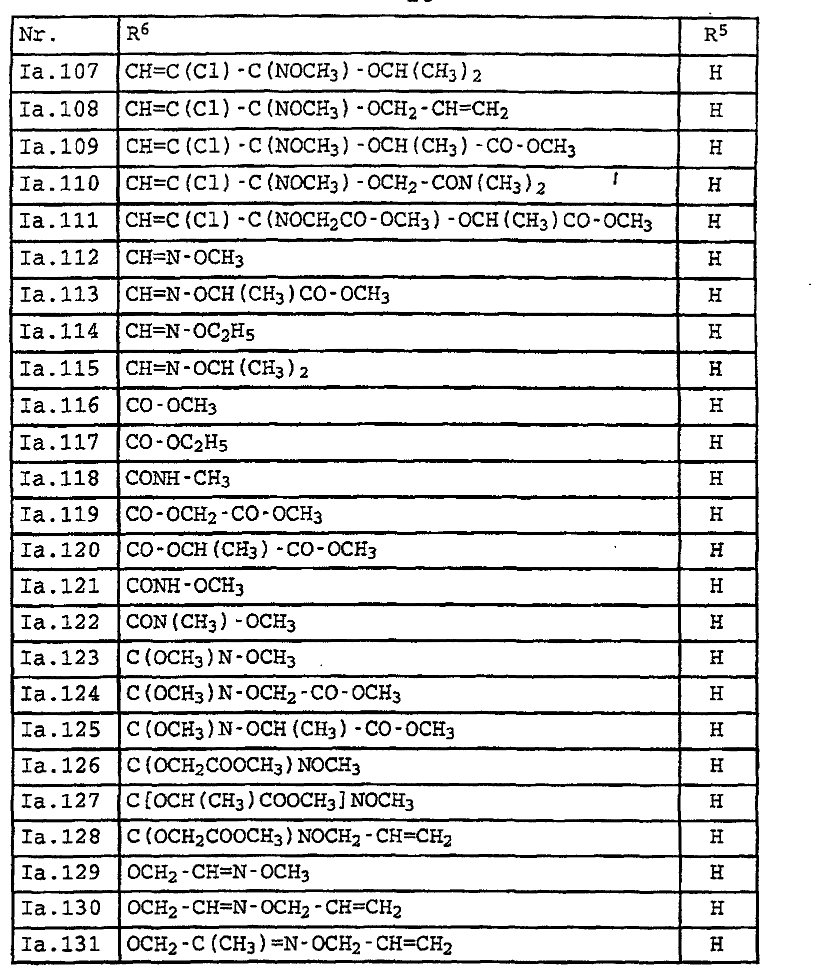

Des weiteren sind die folgenden substituierten 1-Methyl-3-benzyluracile der Formel I besonders bevorzugt:
- die Verbindungen Ib.1 - Ib.131, die sich von den entsprechenden Verbindungen Ia.1 - Ia.131 lediglich dadurch unterscheiden, daß R⁴ Methoxy bedeutet: 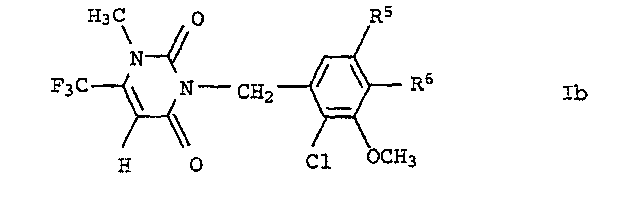
- die Verbindungen Ic.1 - Ic.131, die sich von den entsprechenden Verbindungen Ia.1 - Ia.131 lediglich dadurch unterscheiden, daß R⁴ Cyano bedeutet:

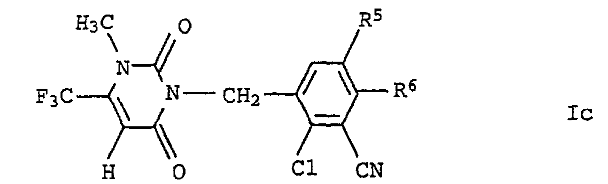

Die substituierten 1-Methyl-3-benzyluracile der Formel I sind auf verschiedene Weise erhältlich, insbesondere nach einem der folgenden Verfahren:

### Verfahren A)

Cyclisierung eines Enaminesters der Formel III oder eines Enamincarboxylates der Formel IV in Gegenwart einer Base: 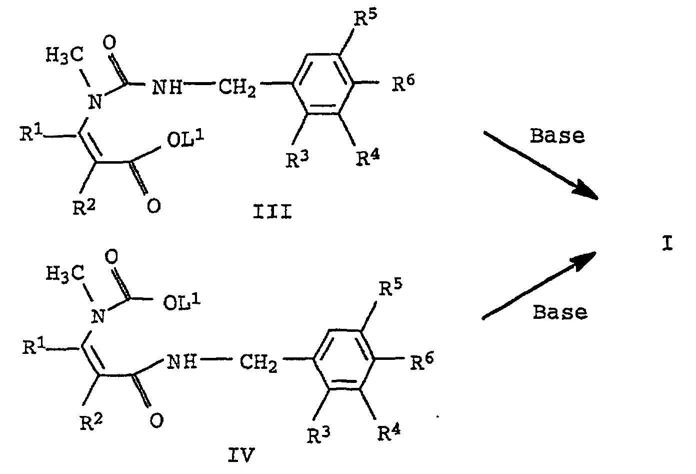
- ¹: L bedeutet niedermolekulares Alkyl, vorzugsweise C₁-C₆-Alkyl, oder Phenyl.
In der Regel cyclisiert man in einem inerten organischen Lösungs- oder Verdünnungsmittel, das aprotisch ist, beispielsweise in einem aliphatischen oder cyclischen Ether wie 1,2-Dimethoxyethan, Tetrahydrofuran und Dioxan, in einem Aromaten wie Benzol und Toluol oder in einem polaren Solvens wie Dimethylformamid und Dimethylsulfoxid. Auch Mischungen aus polarem Solvens und einem Kohlenwasserstoff wie n-Hexan sind geeignet. Je nach Ausgangsverbindung kann sich auch Wasser als Verdünnungsmittel eignen.
Als Basen kommen vorzugsweise Alkalimetallalkoholate, insbesondere die Natriumalkoholate, Alkalimetallhydroxide, insbesondere Natrium- und Kaliumhydroxid, Alkalimetallcarbonate, insbesondere Natrium- und Kaliumcarbonat, und Metallhydride, insbesondere Natriumhydrid, in Betracht. Bei der Verwendung von Natriumhydrid als Base hat es sich als vorteilhaft erwiesen, in einem aliphatischen oder cyclischen Äther, in Dimethylformamid oder in Dimethylsulfoxid zu arbeiten.
Im allgemeinen ist die 0,5- bis zweifache molare Menge an Base, bezogen auf die Menge an III oder IV, für das Gelingen der Reaktion ausreichend.
In der Regel liegt die Reaktionstemperatur zwischen (-78)°C und der Siedetemperatur des jeweiligen Reaktionsgemisches, insbesondere bei (-60) bis 60°C.
Üblicherweise wird das Verfahrensprodukt als Metallsalz erhalten, wobei das Metall dem Kation der verwendeten Base entspricht. Das Salz kann auf an sich bekannte Weise isoliert und gereinigt oder gewünschtenfalls mittels Säure in die freie Verbindung III übergeführt werden.

### Verfahren B)

Methylierung eines 1-H-3-Benzyluracils V in Gegenwart einer Base: 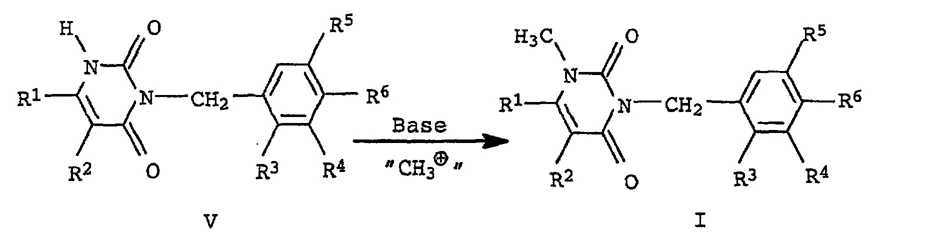 Als Methylierungsmittel kommen beispielsweise Methylhalogenide, vorzugsweise Methylchlorid, -iodid oder -bromid, sowie Dimethylsulfat, Methansulfonat (Methylmesylat), Methylbenzolsulfonat, Methoxy-(p-toluylsulfon) (Methyltosylat), p-Brombenzolsulfonylmethan (Methylbrosylat), Trifluormethansulfonylmethan (Methyltriflat) und Diazomethan in Betracht.
In der Regel arbeitet man in einem inerten organischen Lösungsmittel oder in einem aprotischen Lösungsmittels, z.B. in einem aliphatischen oder cyclischen Äther, vorzugsweise in 1,2-Dimethoxyethan, Tetrahydrofuran oder Dioxan, in einem aliphatischen Keton, vorzugsweise in Aceton, in einem Amid, vorzugsweise in Dimethylformamid, in einem Sulfoxid, vorzugsweise in Dimethylsulfoxid, in einem Harnstoff wie Tetramethylharnstoff und 1,3-Dimethyltetrahydro-2(1H)-pyrimidinon, in einem Carbonsäureester wie Essigsäureethylester, oder in einem halogenierten aliphatischen oder aromatischen Kohlenwasserstoff wie Dichlormethan und Chlorbenzol.
Als Base eignen sich anorganische Basen, z.B. Carbonate wie Natriumcarbonat und Kaliumcarbonat, Hydrogencarbonate wie Natrium- und Kaliumhydrogencarbonat, oder Alkalimetallhydride wie Natriumhydrid und Kaliumhydrid, sowie organische Basen, z.B. Amine wie Triethylamin, Pyridin und N,N-Diethylanilin, oder Alkalimetallalkoholate wie Natriummethanolat, Natriumethanolat und Kalium-tert.-butanolat.
Die Menge an Base und Methylierungsmittel liegt vorzugsweise jeweils bei der 0,5- bis 2-fachen molaren Menge, bezogen auf die Menge an Ausgangsverbindung.
Im allgemeinen liegt die Reaktionstemperatur bei 0°C bis Siedetemperatur des Reaktionsgemisches, insbesondere bei 0 bis 60°C.
Die 1-H-3-Benzyluracile V sind ihrerseits z.B. durch Cyclisierung von Enaminestern VI oder Enamincarboxylaten VII erhältlich: 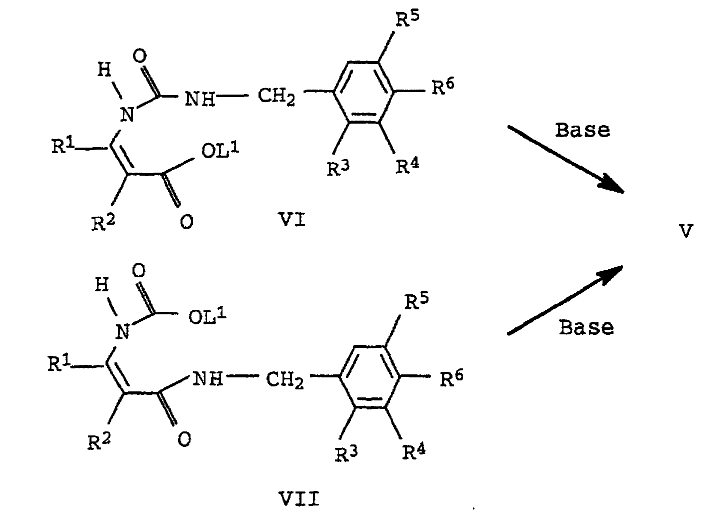 Bezüglich der Lösungs-/Verdünnungsmittel, Basen, Mengenverhältnisse und der Reaktionstemperatur gelten die unter A) gemachten Angaben.
Eine bevorzugte Verfahrensvariante besteht darin, das aus der Cyclisierung von VI oder VII gemäß Verfahren A) erhaltene Salz von I ohne Isolierung aus der Reaktionsmischung - die noch überschüssige Base, z.B. Natriumhydrid, Natriumalkoholat oder Natriumcarbonat, enthalten kann - zu methylieren.

### Verfahren C)

Umsetzung eines 1-Methyl-6-halogenalkyluracils VIII in Gegenwart einer Base mit einem Benzylhalogenid IX: 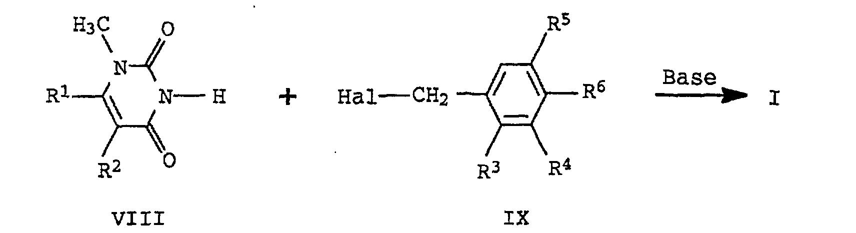 Hal steht für Halogen, besonders bevorzugt Brom.
Geeignete Basen sind beispielsweise Alkalimetallalkoholate wie Natriummethylat, Alkalimetallcarbonate wie Natrium- und Kaliumcarbonat oder Alkalimetallhydride wie Natrium- und Kaliumhydrid.
Es besteht auch die Möglichkeit, VIII mittels Base zunächst in das Alkalimetallsalz zu überführen und dieses dann anschließend mit IX umzusetzen.
Üblicherweise arbeitet man in einem inerten polaren Lösungsoder Verdünnungsmittel, z.B. in Dimethylformamid, N-Methylpyrrolidon, in einem Sulfoxid wie Dimethylsulfoxid, in einem Carbonsäureester wie Ethylacetat, in einem Keton wie Aceton oder in einem Ether wie Diethylether und Tetrahydrofuran.
In allgemeinen liegt die Reaktionstemperatur bei 0°C bis zur Siedetemperatur des Reaktionsgemisches.

### Verfahren D)

Alkylierung eines 1-Methyl-3-benzyluracils der Formel X, in Gegenwart einer Base: 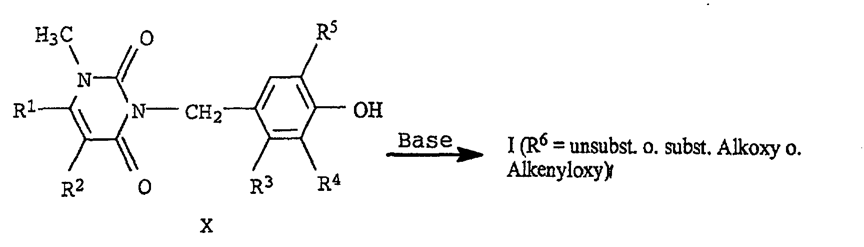 Die Alkylierung kann beispielsweise mit dem Halogenid, vorzugsweise dem Chlorid oder Bromid, dem Sulfat, Sulfonat, vorzugsweise dem Methansulfonat (Mesylat), Benzolsulfonat, p-Toluolsulfonat (Tosylat), p-Brombenzolsulfonat (Brosylat), dem Trifluormethansulfonat (Triflat) oder der Diazoverbindung eines unsubstituierten oder substituierten Alkans oder Alkens vorgenommen werden.
Normalerweise arbeitet man in einem inerten organischen Lösungsmittel, wobei besonders aprotische Lösungsmittel, z.B. aliphatische und cyclische Ether wie 1,2-Dimethoxyethan, Tetrahydrofuran und Dioxan, aliphatische Ketone wie Aceton, Amide wie Dimethylformamid, Sulfoxide wie Dimethylsulfoxid, Harnstoffen wie Tetramethylharnstoff und 1,3-Dimethyltetrahydro-2(1H)-pyrimidinon, Carbonsäureester wie Essigsäureethylester, oder halogenierte aliphatische oder aromatische Kohlenwasserstoffe wie Dichlormethan und Chlorbenzol, in Betracht kommen.
Als Base eignen sich sowohl anorganische Basen, z.B. Alkalimetallcarbonate wie Natriumcarbonat und Kaliumcarbonat, Alkalimetallhydrogencarbonate wie Natrium- und Kaliumhydrogencarbonat, oder Alkalimetallhydride wie Natriumhydrid und Kaliumhydrid, als auch organische Basen, z.B. Amine wie Triethylamin, Pyridin und N,N-Diethylanilin, oder Alkalimetallalkoholate wie Natriummethanolat, -ethanolat und Kalium-tert.-butanolat.
Die Menge an Base und Alkylierungsmittel liegt vorzugsweise bei der 0,5- bis 2-fachen molaren Menge, bezogen auf die Menge an X.
Im allgemeinen empfiehlt sich eine Reaktionstemperatur von 0°C bis zur Siedetemperatur des Reaktionsgemisches, insbesondere von 0 bis 60°C.
Mit dieser Methode lassen sich auch Verbindungen I mit R⁴ = C₁-C₄-Alkoxy aus den entsprechenden Hydroxy-Vorprodukten herstellen.

### Verfahren E)

Substitution von Halogenid durch Cyanid: 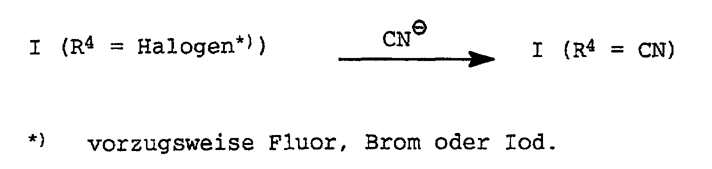 Geeignete Cyanide sind insbesondere Metallcyanide, z.B. die Alkalimetallcyanide wie Lithium-, Natrium- und Kaliumcyanid, die Erdalkalimetallcyanide wie Magnesiumcyanid, oder auch Übergangsmetallcyanide wie Kupfercyanid.
Üblicherweise arbeitet man in einem Ether wie Tetrahydrofuran, Dioxan und 1,2-Dimethoxyethan, oder in einem aprotischen, polaren Lösungsmittels, z.B. einem Alkylnitril wie Aceto-, Propio- und Butyronitril, einem Alkylharnstoff wie N,N,N',N'-Tetramethylhamstoff, einem offenkettigen oder cyclischen Dialkylamid wie Dimethylformamid, N-Methyl-2-pyrrolidon, 1,2-Dimethyl-imidazolidin-2-on und 1,2-Dimethyl-3,4,5,6-tetrahydro-2(1H)-pyrimidinon, einem Dialkylsulfoxid wie Dimethylsulfoxid, oder in Hexamethylphosphorsäuretriamid.
Nach den bisherigen Erkenntnissen kann sich die Gegenwart eines Katalysators vorteilhaft auf den Reaktionsverlauf auswirken. Brauchbare Katalysatoren sind z.B. Übergangsmetalle und deren Komplexe oder Salze, z.B. Verbindungen des Kupfers wie Kupfer-(I)-Chlorid, -iodid, -cyanid, oder des Nickels wie Nickel-bis-triphenylphospin-dibromid.
Auf analoge Weise können auch die Ausgangsverbindungen V mit R⁴ = Halogen in die entsprechenden Verbindungen V mit R⁴ = CN übergeführt werden. Dabei ist es jedoch empfehlenswert, in Gegenwart einer Base zu arbeiten, wobei insbesondere schwach nucleophile Basen in Betracht kommen, und zwar sowohl anorganische Basen, z.B. Alkalimetallcarbonate wie Natrium- und Kaliumcarbonat, Alkalimetallhydrogencarbonate wie Natriumund Kaliumhydrogencarbonat, oder Alkalimetallhydride wie Natriumhydrid und Kaliumhydrid, als auch organische Basen, z.B. Amine wie Triethylamin, Pyridin und N,N-Diethylanilin.
Die Mengenverhältnisse sind normalerweise nicht kritisch. Im allgemeinen ist die etwa ein- bis 10-fache Menge an Cyanid und Base, bezogen auf die Menge an Ausgangsverbindung I oder V, ausreichend.
Die Reaktionstemperatur liegt üblicherweise bei 50 bis 250°C; zur Erhöhung der Selektivität der Reaktion kann es aber auch empfehlenswert sein, bei tieferen Temperaturen, insbesondere bei etwa 20°C, zu arbeiten.
Bezüglich verschiedener Ausführungsformen dieser Umsetzung sei auf Houben-Weyl, "Methoden der Organischen Chemie", Georg Thieme Verlag, 4. Auflage, Stuttgart 1985, Bd. E5, S. 1444ff. sowie auf die dort angegebene Literatur verwiesen.

Weitere zur Herstellung der substituierten 1-Methyl-3-benzyluracile I brauchbare Methoden können Houben-Weyl, Methoden der Org. Chemie, 4. Auflage, Bd. E3, S. 362 ff., entnommen werden.

### Verfahren F)

Olefinierung von Verbindungen XI: 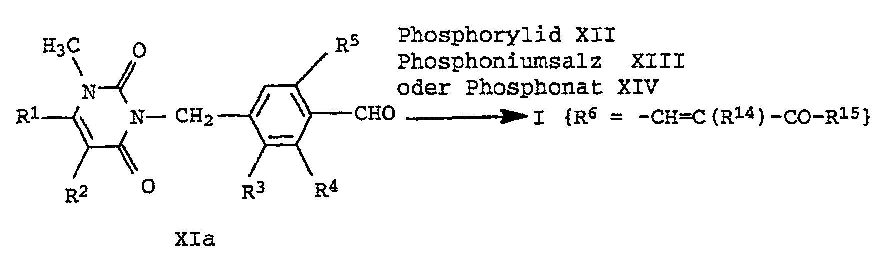 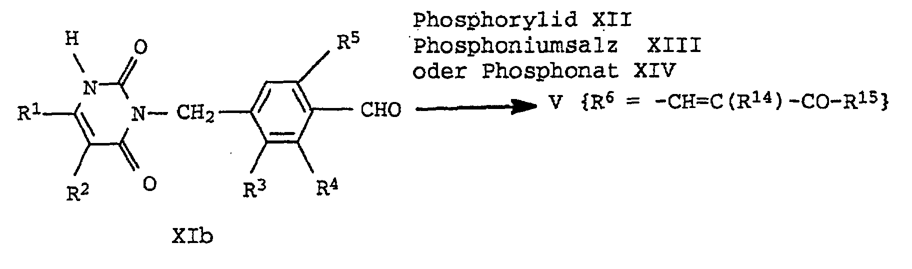 Die Reaktion kann mit den folgenden Phosphoryliden XII, Phosphoniumsalzen XIII und Phosphonaten XIV durchgeführt werden:
Phosphorylide XII: R₃P=C(R¹⁴)-CO-R¹⁵
Phosphoniumsalze XIII: R₃P^{⊕}-CH(R¹⁴)-CO-R¹⁵ Hal^{⊖}
Phosphonate XIV: (RO)₂PO-CH(R¹⁴)-CO-R¹⁵
Die Reste R am Phosphor können gleich oder verschieden sein und stehen beispielsweise für verzweigte oder unverzweigte C₁-C₈-Alkylgruppen, C₅- oder C₆-Cycloalkylgruppen und insbesondere für Phenyl, das weitere (für die Umsetzung inerte Substituenten, beispielsweise C₁-C₄-Alkyl wie Methyl, Ethyl und tert.-Butyl, C₁-C₄-Alkoxy wie Methoxy oder Halogen wie Fluor, Chlor und Brom) tragen kann. Bevorzugt sind unsubstituierte Phenylreste, da der für die Herstellung der Phosphorylide XII und Phosphoniumsalze XIII verwendete Ausgangsstoff Triphenylphosphin besonders kostengünstig ist und bei den Umsetzungen zudem das sehr reaktionsträge und gut abtrennbare, feste Triphenylphosphinoxid entsteht.
Zur Herstellung der Phosphonate XIV eignen sich beispielsweise die in Houben-Weyl, Methoden der Organischen Chemie, Bd. E2, 1982, S. 345ff., beschriebenen Methoden.
Als Lösungsmittel kommen inerte organische Lösungsmittel, z.B. Aromaten wie Toluol und o-, m-, p-Xylol, Ether wie 1,2-Dimethoxyethan, Diethylether, Tetrahydrofuran und Dioxan, polare organische Lösungsmittel wie Dimethylformamid und Dimethylsulfoxid, oder Alkohole wie Methanol, Ethanol und Isopropanol, in Betracht.
Bei der Olefinierung von XI mit einem Phosphoniumsalz XIII oder einem Phosphonat XIV arbeitet man in Gegenwart einer Base, wobei Alkalimetallalkyle wie n-Butyllithium, Alkalimetallhydride und -alkoholate wie Natriumhydrid, Natriumethanolat und Kalium-tert.-butanolat, sowie Alkalimetall- und Erdalkalimetallhydroxide wie Calciumhydroxid, besonders gut geeignet sind.
Für eine vollständige Umsetzung werden alle Reaktionspartner in etwa stöchiometrischen Mengen eingesetzt; bevorzugt verwendet man jedoch einen Überschuß an Base, bis etwa 10 mol-%.
Im allgemeinen liegt die Reaktionstemperatur bei (-40) bis 150°C.
Die Verbindungen der Formel XII, XIII und XIV sind bekannt oder lassen sich in auf bekannte Weise darstellen (vgl. z.B. Houben-Weyl, Methoden d. Org. Chemie, Bd. E1, S. 636 ff., Georg Thieme Verlag, Stuttgart 1982, Chem. Ber. 95, 3993 1962) oder Houben-Weyl, Methoden d. Org. Chemie, Bd. E2, S. 345ff., Georg Thieme Verlag, Stuttgart 1982).
Eine weitere Möglichkeit zur Darstellung von 1-Methyl-3-benzyluracilen I, wobei R⁶ für -CH=C(R¹⁴)-CO-R¹⁵ steht, besteht in der an sich bekannten Aldolkondensation. Hierfür geeignete Bedingungen sind z.B. Nielsen, Org. React. 16, 1ff. (1968), zu entnehmen.
Als weitere Methode zur Synthese von Verbindungen der Formel I, wobei R⁶ für -CH=C(R¹⁴)-CO-R¹⁵ steht und R¹⁴ Wasserstoff bedeutet, kommen sowohl die Knoevenagel-Kondensation als auch die Perkin-Kondensation in Betracht. Geeignete Bedingungen sind z.B. aus Org. React. 1967, 15, 204ff. (Knoevenagel) bzw. Johnson, Org. React. 1, 1942, 210ff. (Perkin), ersichtlich.
Verbindungen, in denen R¹⁵ -N(R²³)R²⁴ bedeutet, lassen sich z.B. in an sich bekannter Weise dadurch herstellen, daß man entsprechende Verbindungen, in denen R¹⁵ für Hydroxy steht, in ihre Säurehalogenide (Halogen anstelle von R¹⁵) überführt und die Verfahrensprodukte anschließend mit einem Amin H-N(R²³)R²⁴ oder mit einem reaktiven Derivat dieser Verbindung umsetzt.

### Verfahren G)

Umsetzung von Verbindungen XI mit Hydroxylaminen: 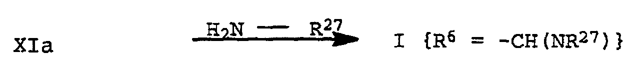 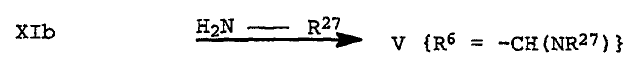 Die Umsetzung erfolgt normalerweise in einem inerten organischen Lösungs- oder Verdünnungsmittel, z.B. in einem Aromaten wie Toluol und Xylol, in einem chlorierten Kohlenwasserstoff wie Dichlormethan, Chloroform und Chlorbenzol, in einem Ether wie Diethylether, 1,2-Dimethoxyethan und Tetrahydrofuran, in einem Alkohol wie Methanol und Ethanol, oder in einem Gemisch der genannten Lösungsmittel.
Liegen die Hydroxylamine H₂N-R²⁷ als Salze, z.B. als Hydrochloride oder Oxalate vor, so ist zu ihrer Freisetzung die Zugabe einer Base wie vorzugsweise Natriumcarbonat, Kaliumcarbonat, Natriumhydrogencarbonat, Triethylamin und Pyridin empfehlenswert.
Das entstehende Reaktionswasser kann gegebenenfalls destillativ oder mit Hilfe eines Wasserabscheiders aus dem Reaktionsgemisch entfernt werden.
Üblicherweise liegt die Reaktionstemperatur bei (-30) bis 150°C, bevorzugt 0 bis 130°C.

### Verfahren H)

Metallkatalysierte Olefinkupplung mit einem Phenylhalogenid der Formel XV: 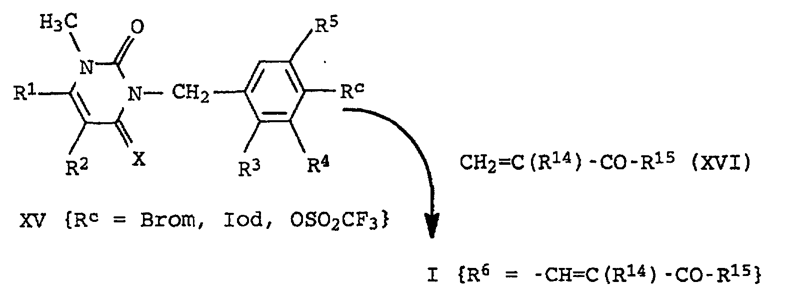 Die Bedingungen dieser Heck- oder Heck-ähnlichen Reaktion sind dem Fachmann an sich bekannt (vgl. z.B. Comprehensive Organic Chemistry) und lassen sich analog auf obige Reaktion anwenden.

Die Enaminester der Formel III sind neu. Ihre Herstellung und die der Enaminester VI kann nach an sich bekannten Methoden erfolgen, z.B. nach einem der folgenden Verfahren:

### Verfahren K)

Umsetzung eines 3-Amino-prop-2-ensäureesters XVII mit einem Benzylisocyanat XVIII in Gegenwart einer Base: 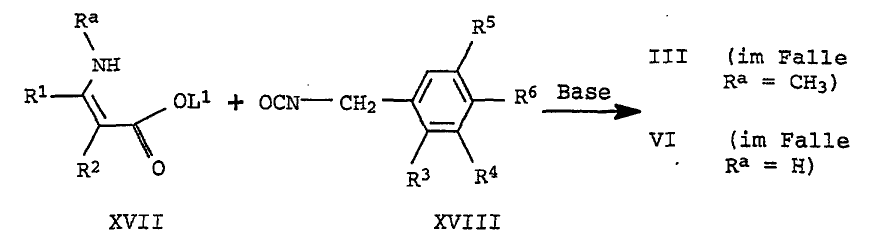
- R^{a}: steht für Wasserstoff oder Methyl.
Als 3-Amino-prop-2-ensäureester XVII hat sich bisher der Ethylester besonders bewährt, jedoch kann auch jeder andere Ester, vorzugsweise die Alkylester, verwendet werden.
Die Umsetzung erfolgt zweckmäßig in Gegenwart eines im wesentlichen wasserfreien aprotischen organischen Lösungsoder Verdünnungsmittels, beispielsweise eines aliphatischen oder cyclischen Ethers wie Diethylether, 1,2-Dimethoxyethan, Tetrahydrofuran und Dioxan, eines aliphatischen oder aromatischen Kohlenwasserstoffs wie n-Hexan, Benzol, Toluol und den Xylolen, eines halogenierten, aliphatischen Kohlenwasserstoffs wie Methylenchlorid, Chloroform, Tetrachlorkohlenstoff, 1,2-Dichlorethan und Chlorbenzol, eines aprotischen, polaren Lösungsmittel wie Dimethylformamid, Hexamethylphosphorsäuretriamid und Dimethylsulfoxid, oder eines Gemisches aus den genannten Solventien.
Gewünschtenfalls kann auch in Gegenwart einer Metallhydridbase wie Natrium- und Kaliumhydrid, eines Alkalimetall- oder Erdalkalimetallalkoholates wie Natriummethanolat, -ethanolat und Kalium-tert.-butanolat, oder einer organischen tertiären Base wie Triethylamin und Pyridin gearbeitet werden, wobei die organische Base gleichzeitig als Lösungsmittel dienen kann.
Zweckmäßig setzt man die Ausgangsverbindungen in stöchiometrischen Mengen ein oder man arbeitet mit einem geringen Überschuß der einen oder anderen Komponente bis etwa 10 mol-%. Beim Arbeiten ohne Lösungsmittel in Gegenwart einer organischen Base empfiehlt es sich, letztere in einem größeren Überschuß einzusetzen.
Normalerweise ist eine Reaktionstemperatur von (-80) bis 50°C, insbesondere (-60) bis 30°C ausreichend.
In einer besonders bevorzugten Ausführungsform wird der erhaltene Enaminester mit überschüssiger Base direkt (d.h. "in situ") in das entsprechende Wertprodukt III oder VI übergeführt, dessen Reinigung dann mittels üblicher Trennverfahren wie Kristallisation und Chromatographie erfolgen kann.

### Verfahren L)

Umsetzung eines β-Ketoesters XIX mit einem Benzylharnstoff XX: 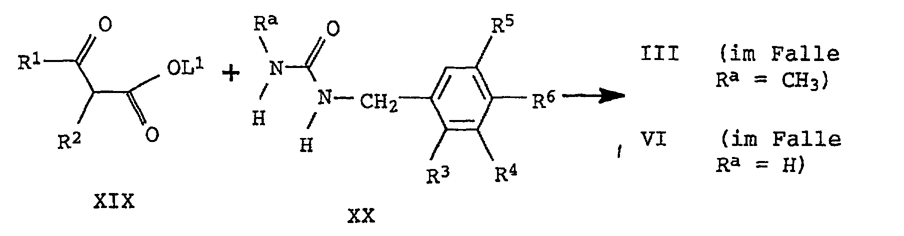 Vorzugsweise arbeitet man im wesentlichen wasserfrei in einem inerten Lösungs- oder Verdünnungsmittel, besonders bevorzugt in Gegenwart eines sauren oder basischen Katalysators.
Als Lösungs- oder Verdünnungsmittel kommen insbesondere mit Wasser azeotrop mischbare organische Lösungsmittel, beispielsweise Aromaten wie Benzol, Toluol und die Xylole, halogenierte Kohlenwasserstoffe wie Methylenchlorid, Chloroform, Tetrachlorkohlenstoff und Chlorbenzol, aliphatische und cyclische Ether wie 1,2-Dimethoxyethan, Tetrahydrofuran und Dioxan, oder Cyclohexan, aber auch Alkohole wie Methanol und Ethanol, in Betracht.
Als saure Katalysatoren eignen sich bevorzugt starke Mineralsäuren wie Schwefelsäure und Salzsäure, Phosphor enthaltende Säuren wie Orthophosphorsäure und Polyphosphorsäure, organische Säuren wie p-Toluolsulfonsäure sowie saure Kationenaustauscher wie "Amberlyst 15" (Fa. Fluka).
Als basische Katalysatoren eignen sich z.B. Metallhydride wie Natriumhydrid sowie besonders bevorzugt Metallalkoholate wie Natriummethanolat und Ethanolat.
Zweckmäßig setzt man β-Ketoester XIX und den Benzylharnstoff XX in etwa stöchiometrischen Mengen um oder man arbeitet mit einem geringen Überschuß der einen oder anderen Komponente, bis etwa 10 mol-%.
Normalerweise ist es ausreichend, die halbe bis zweifache molare Menge an Katalysator, bezogen auf die Menge einer der Ausgangsverbindungen, einzusetzen.
Im allgemeinen erfolgt die Reaktionsführung bei einer Temperatur von 60 bis 120°C, zur raschen Entfernung von entstehendem Wasser vorzugsweise bei der Siedetemperatur des Reaktionsgemisches.

### verfahren M)

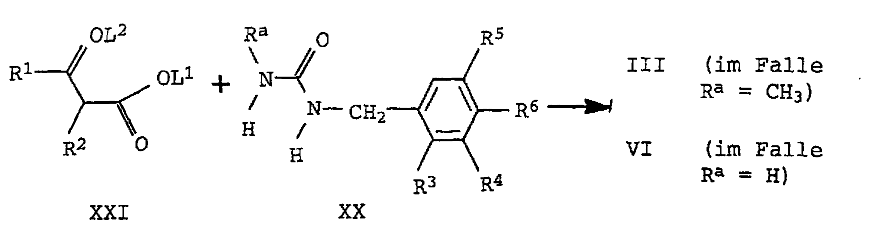

- L²: steht für C₁-C₆-Alkyl oder Phenyl.
Diese Umsetzung kann in einem inerten, mit Wasser mischbaren, organischen Lösungsmittel, beispielsweise einem aliphatischen oder cyclischen Ether wie 1,2-Dimethoxyethan, Tetrahydrofuran und Dioxan, oder einem niederen Alkohol, insbesondere Ethanol, durchgeführt werden, wobei die Reaktionstemperatur normalerweise bei 50 bis 100°C, vorzugsweise bei der Siedetemperatur des Reaktionsgemisches, liegt.
Die Reaktion kann jedoch auch in einem aromatischen Verdünnungsmittel wie Benzol, Toluol und den Xylolen durchgeführt werden, wobei in diesem Fall der Zusatz entweder eines sauren Katalysators wie Salzsäure und p-Toluolsulfonsäure oder einer Base, z.B. eines Alkalimetallalkoholates wie Natriummethanolat und Natriumethanolat, empfehlenswert ist. Auch bei dieser Verfahrensvariante liegt die Reaktionstemperatur normalerweise bei 50 bis 100°C, bevorzugt jedoch bei 60 bis 80°C.
Bezüglich der Mengenverhältnisse gelten die Angaben für Methode L).

Die Enamincarboxylate der Formel IV sind ebenfalls neu; auch sie - und die Enamincarboxylate VII - können auf an sich bekannte Weise hergestellt werden, beispielsweise aus einem Benzylamin der Formel XXI nach folgendem allgemeinen Reaktionsschema N): 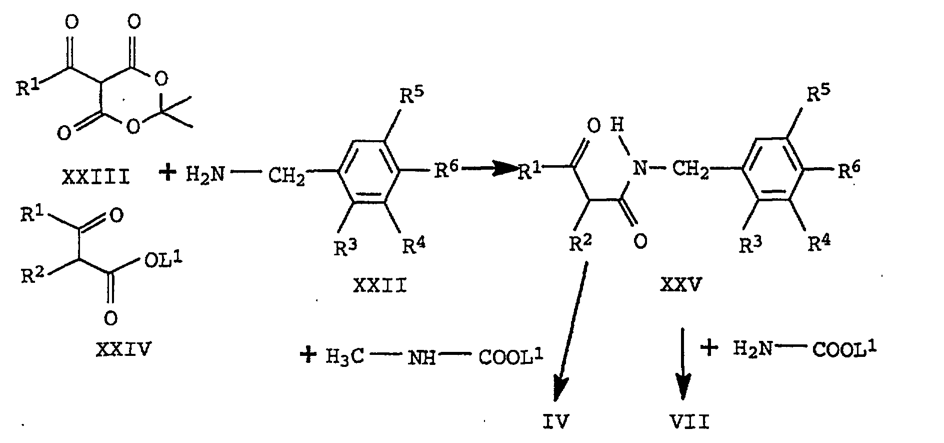 Die Umsetzung von XXII mit XXIII erfolgt vorzugsweise in einem wasserfreien inerten aprotischen Lösungsmittel, beispielsweise in einem halogenierten Kohlenwasserstoff wie Methylenchlorid, Chloroform, Tetrachlorkohlenstoff und Chlorbenzol, einem aromatischen Kohlenwasserstoff wie Benzol, Toluol und den Xylolen, oder einem aliphatischen oder cyclischen Ether wie Diethylether, Dibutylether, 1,2-Dimethoxyethan, Tetrahydrofuran und Dioxan.
Die Reaktionstemperatur liegt bei dieser Umsetzung (von XXII mit XXIII) im allgemeinen bei etwa 70 bis 140°C, insbesondere bei 100 bis 120°C.
Bei der Umsetzung von XXII mit XXIV handelt es sich um eine Aminolyse, die in der Regel entweder ohne Lösungsmittel [vgl. z.B. J. Soc. Dyes Col. 42, 81 (1926), Ber. 64, 970 (1931); Org. Synth., Coll. Vol. IV, 80 (1963) und J. Am. Chem. Soc. 70, 2402 (1948)] oder in einem inerten wasserfreien Lösungs-/Verdünnungsmittel, insbesondere in einem aprotischen Solvens, beispielsweise in einem Aromaten wie Toluol und den Xylolen, oder einem halogenierten Aromaten wie Chlorbenzol, durchgeführt wird.
Hierbei empfiehlt sich das Arbeiten in Gegenwart eines basischen Katalysators, beispielsweise eines höher siedenden Amins [siehe z. B. Helv. Chim. Acta 11, 779 (1928) und U.S. 2,416,738] oder von Pyridin.
Vorzugsweise liegt die Reaktionstemperatur bei ca. 20 bis 160°C, insbesondere bei 80°C bis zur Siedetemperatur des Reaktionsgemisches oder des basischen Katalysators.
Zweckmäßigerweise setzt man die Ausgangsverbindungen jeweils in etwa stöchiometrischen Mengen um oder man arbeitet mit einem geringen Überschuß der einen oder anderen Komponente, bis etwa 10 mol-%. Arbeitet man in Gegenwart eines basischen Katalysators, so wird dieser normalerweise in der halben bis zur zweifachen molaren Menge, bezogen auf die Menge eines der Edukte, eingesetzt.
Die anschließende Umsetzung der so hergestellten Verbindungen der Formel XXV mit einem Amin H₂N-COOL¹ oder H₃C-NH-COOL¹ wird vorteilhaft in einem weitgehend wasserfreien Lösungs-/Verdünnungsmittel bei Normaldruck durchgeführt, besonders bevorzugt in Gegenwart eines sauren Katalysators.
Als Lösungs-/Verdünnungsmittel kommen insbesondere mit Wasser azeotrop mischbare organische Flüssigkeiten, beispielsweise Aromaten wie Benzol, Toluol und die Xylole, oder halogenierte Kohlenwasserstoffe wie Tetrachlorkohlenstoff und Chlorbenzol, in Betracht.
Geeignete Katalysatoren sind insbesondere starke Mineralsäuren wie Schwefelsäure, organische Säuren wie p-Toluolsulfonsäure, Phosphor enthaltende Säuren wie Orthophosphorsäure und Polyphosphorsäure oder saure Kationenaustauscher wie "Amberlyst 15" (Fa. Fluka).
Im allgemeinen liegt die Reaktionstemperatur bei etwa 70 bis 150°C; zur raschen Entfernung des entstehenden Reaktionswassers arbeitet man jedoch zweckmäßigerweise bei der Siedetemperatur des jeweiligen Reaktionsgemisches.

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Die Aufarbeitung der Reaktionsgemische erfolgt in der Regel nach an sich bekannten Methoden, beispielsweise durch Verdünnen der Reaktionslösung mit Wasser und anschließender Isolierung des Produktes mittels Filtration, Kristallisation oder Lösungsmittelextraktion, oder
durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die substituierten 1-Methyl-3-benzyluracile I können bei der Herstellung als Isomerengemische anfallen, die jedoch gewünschtenfalls nach den hierfür üblichen Methoden wie Kristallisation oder Chromatographie, auch an einem optisch aktiven Adsorbat, in die reinen Isomeren getrennt werden können. Reine optisch aktive Isomere lassen sich vorteilhaft aus entsprechenden optisch aktiven Ausgangsprodukten herstellen.

Landwirtschaftlich brauchbare Salze der Verbindungen I können durch Reaktion mit einer Base des entsprechenden Kations, vorzugsweise einem Alkalimetallhydroxid oder -hydrid, oder durch Reaktion mit einer Säure des entsprechenden Anions, vorzugsweise der Chlorwasserstoffsäure, Bromwasserstoffsäure, Schwefelsäure, Phosphorsäure oder Salpetersäure, gebildet werden.

Salze von I, deren Metallion kein Alkalimetallion ist, können auch durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Des weiteren eignen sich die substituierten 1-Methyl-3-benzyluracile I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe kommen im Wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die substituierten 1-Methyl-3-benzyluracile als solche oder in einem Öl oder Lösungsmittel gelöst, mittels Netz-, Haft-, Dispergieroder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Im allgemeinen enthalten die Formulierungen etwa 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die folgenden Formulierungsbeispiele verdeutlichen die Herstellung solcher Zubereitungen:
I. 20 Gewichtsteile der Verbindung Nr. Ia.4 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
II. 3 Gewichtsteile des Wirkstoffs Nr. Ia.46 werden mit 97 Gewichtsteilen feinteiligem Kaolin vermischt. Man erhält auf diese Weise ein Stäubemittel, das 3 Gew.-% des Wirkstoffs enthält.
III. 1 Gewichtsteil der Verbindung Nr. Ia.131 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl; BASF AG) besteht. Man erhält ein stabiles Emulsionskonzentrat.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die substituierten 1-Methyl-3-benzyluracile I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1

### 3-(2,3-Dichlor-4-isopropoxybenzyl)-2,4-dioxo-1-methyl-6-trifluormethyl-1,2,3,4-tetrahydropyrimidin (Verbindung Nr. Ia.4)

Zu einer Lösung von 1,8 g 3-(2,3-Dichlor-4-isopropoxybenzyl)-2,4-dioxo-1H-6-trifluormethyl-1,2,3,4-tetrahydropyrimidin in 50 ml Dimethylformamid wurden bei ca. 20°C 0,7 g Kaliumcarbonat und 0,7 g Methyliodid gegeben, wonach man 18 Std. rührte. Zur Aufarbeitung wurden 150 ml Eiswasser in die Reaktionsmischung gegeben. Anschließend trennte man das feste Wertprodukt ab.
Ausbeute: 1,4 g; Fp.: 167-168°C.

### Vorstufe 1:

### 2-(2,3-Dichlorphenoxy)propan

Zu einer Mischung aus 100 g 2,3-Dichlorphenyl, 92,4 g Kaliumcarbonat und 400 ml Dimethylformamid wurden 62,9 ml 2-Brompropan getropft. Dann rührte man 16 Std. bei ca. 20°C und anschließend noch 8 Std. bei 60°C. Nach filtrieren des Reaktionsgemisches wurde eingeengt. Den Rückstand nahm man in Methylenchlorid auf. Die organische Phase wurde mit Wasser gewaschen, dann über Natriumsulfat getrocknet und schließlich eingeengt. Ausbeute: 102 g (Öl; analysenrein).

### Vorstufe 2:

### 2-(2,3-Dichlor-4-chlormethyl-phenoxy)propan

In eine Mischung aus 228 g 2-(2,3-Dichlorphenoxy)propan, 600 ml Eisessig und 220 ml 30 Gew.%iger wäßriger Formaldehyd-Lösung wurde 13 Std. lang Chlorwasserstoff-Gas eingeleitet. Anschließend rührte man noch 12 Std. bei ca. 20°C, wonach das Lösungsmittel entfernt wurde. Den Rückstand löste man in Diethylether. Die Etherphase wurde mit Wasser gewaschen, dann über Natriumsulfat getrocknet und schließlich eingeengt. Ausbeute: 254 g (Öl; analysenrein).

### Vorstufe 3:

### N-(2,3-Dichlor-4-isopropoxy-benzyl)-3,4,5,6-tetrahydrophthalimid

Zu einer Lösung von 9,26 g Phthalimid-Kalium in 250 ml Dimethylformamid wurden bei 50°C 12,5 g 2-(2,3-Dichlor-4-chlormethylphenoxy)propan gegeben. Anschließend erhitzte man 3 Std. lang auf 120°C, wonach man einengte. Der Rest wurde auf Wasser gegossen. Aus der wäßrigen Phase wurde das Produkt dann mit Methylenchlorid extrahiert. Die organischen Phase wurde schließlich über Natriumsulfat getrocknet und anschließend eingeengt. Ausbeute: 17 g (Öl; aus Petrolether farblose Kristalle mit einem Schmelzpunkt von 103-105°C.

### Vorstufe 4:

### 2-(2,3-Dichlor-4-aminomethyl-phenoxy)propan

Zu einer Lösung von 6,5 g N-(2,3-Dichlor-4-isopropoxy-benzyl)-3,4,5,6-tetrahydrophthalimid in 150 ml Ethanol wurden 1,75 g Hydrazinhydrat gegeben, wonach man 2 Std. auf 90°C erhitzte. Danach wurde die Reaktionsmischung filtriert. Nach einengen des Filtrats erhielt man ein Öl, das in Essigsäureethylester aufgenommen wurde. Aus der entstandenen Lösung extrahierte man das Produkt mit verdünnter Salzsäure. Die Säure-Lösung wurde anschließend bis zu einem pH-Wert von 10 mit Natronlauge versetzt, wonach man das Produkt wieder mit Essigsäureethylester extrahierte. Die Ester-Phase wurde schließlich über Natriumsulfat getrocknet und eingeengt. Ausbeute: 1,5 g.

### Vorstufe 5:

### 2-(2,3-Dichlor-4-isocyanatomethyl-phenoxy)propan

Zu einer Lösung von 65 g 2-(2,3-Dichlor-4-aminomethyl-phenoxy)-propan in 400 ml Toluol wurden 59 g Diphosgen gegeben. Anschließend erwärmte man langsam auf 110°C. Nach 15 Std. rühren bei dieser Temperatur wurde das Reaktionsgemisch abgekühlt und eingeengt. Ausbeute: 70 g (Öl).

### Vorstufe 6:

### 3-(2,3-Dichlor-4-isopropoxybenzyl)-2,4-dioxo-1H-6-trifluormethyl-1,2,3,4-tetrahydropyrimidin

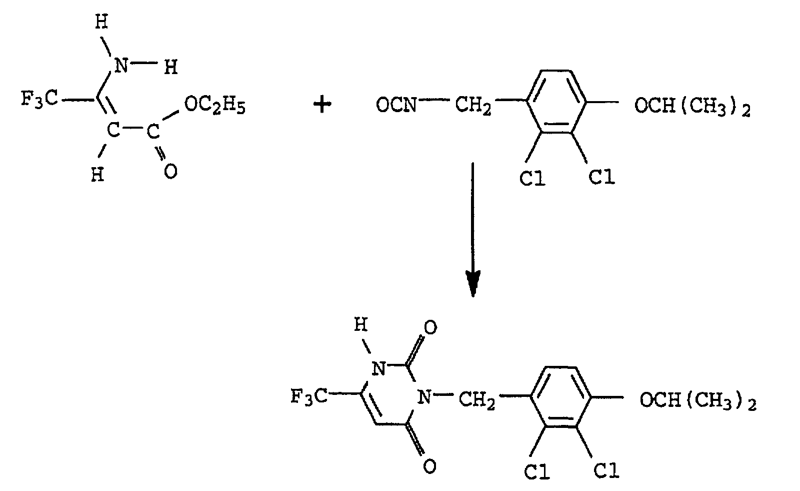

Zu 3 g Natriumhydrid (85 gew.-%ig in Mineralöl) in 200 ml Tetrahydrofuran wurde bei 0°C eine Lösung von 15 g F₃C-C(NH₂)=CH-CO-OC₂H₅ in 25 ml Dimethylformamid getropft. Nach 30 Minuten rühren bei 0°C kühlte man die Mischung auf (-30)°C und tropfte bei dieser Temperatur langsam eine Lösung von 359 g 2-(2,3-Dichlor-4-isocyanatomethyl-phenoxy)propan in 25 ml Tetrahydrofuran zu. Anschließend wurde das Reaktionsgemisch auf ca. 20°C erwärmt, wonach man weitere 2 Std. rührte. Dann wurde das Lösungsmittel abdestilliert. Den Rückstand nahm man in 200 ml Essigsäureethylester auf. Die Ester-Lösung wurde mit verdünnter Salzsäure gewaschen, dann über Natriumsulfat getrocknet und schließlich eingeengt. Die Reinigung des erhaltenen Öls erfolgte mittels Chromatographie an Kieselgel (Eluens: Methylenchlorid).
Ausbeute: 13 g.

### Beispiel 2

### 3-(2,3-Dichlor-4-hydroxybenzyl)-2,4-dioxo-1-methyl-6-trifluormethyl-1,2,3,4-tetrahydropyrimidin (Vorprodukt der Formel X)

Zu einer Lösung von 0,6 g 3-(2,3-Dichlor-4-isopropoxybenzyl)-2,4-dioxo-1-methyl-6-trifluormethyl-1,2,3,4-tetrahydropyrimidin (vgl. Bsp. 1) in 15 ml Methylenchlorid wurden bei ca. 20°C 0,28 g Bortribromid getropft. Nach 2 Std. Rühren versetzte man die Reaktionsmischung mit 100 ml Eiswasser. Anschließend wurde erst noch 30 Minuten gerührt, bevor man das entstandene feste Wertprodukt abtrennte und trocknete. Ausbeute: 0,3 g (analysenrein); Fp.: 241-242°C.

### Beispiel 3

### 3-(2,3-Dichlor-4-[1-(methoxycarbonyl)ethoxy]benzyl)-2,4-dioxo-1-methyl-6-trifluormethyl-1,2,3,4-tetrahydropyrimidin (Verbindung Nr. Ia.20)

Zu einer Lösung von 3,6 g 3-(2,3-Dichlor-4-hydroxy-benzyl)-2,4-dioxo-1-methyl-6-trifluormethyl-1,2,3,4-tetrahydropyrimidin in 50 ml Dimethylformamid wurden bei ca. 20°C 1,3 ml 2-Brompropionsäuremethylester gegeben, wonach man 18 Std. rührte. Anschließend wurde das Lösungsmittel abdestilliert. Den Rückstand nahm man in 50 ml Methylenchlorid auf. Die erhaltene organische Phase wurde zunächst zweimal mit Wasser gewaschen, dann über Natriumsulfat getrocknet und schließlich eingeengt. Die Reinigung des erhaltenen Rohprodukts erfolgte mittels Kieselgelchromatographie (Laufmittel: Methylenchlorid). Ausbeute: 0,7 g (Öl).

### Beispiel 4

### 3-[2,3-Dichlor-4-(hydroxycarbonyl-methoxy)benzyl]-2,4-dioxo-1-methyl-6-trifluormethyl-1,2,3,4-tetrahydropyrimidin (nicht mehr erfindungsgemäß)

Zu einer Lösung von 3-[2,3-Dichlor-4-(tert.-butoxycarbonylmethoxy)benzyl]-2,4-dioxo-1-methyl-6-trifluormethyl-1,2,3,4-tetrahydropyrimidin in 30 ml Methylenchlorid wurden bei ca. 20°C 30 ml Trifluoressigsäure gegeben. Nach 2 Std. rühren destillierte man das Lösungsmittel ab. Der Rückstand wurde mit 100 ml Wasser versetzt. Anschließend extrahierte man das Wertprodukte mit dreimal 50 ml Methylenchlorid. Die vereinigten organischen Phasen wurden dann noch über Natriumsulfat getrocknet und schließlich eingeengt. Die Reinigung des Rohprodukts erfolgte durch Lösen in wenig Diethylether und Fällung mittels Petrolether.
Ausbeute: 2,5 g; Fp.: 140°C (Zers.).

### Beispiel 5

### 3-(2,3-Dichlor-4-[1-(methoxycarbonyl)ethoxycarbonyl-methoxy]-benzyl)-2,4-dioxo-1-methyl-6-trifluormethyl-1,2,3,4-tetrahydropyrimidin (Verbindung Nr. Ia.46)

Zu einer Mischung aus 0,23 g Milchsäuremethylester, 0,25 g Triethylamin und 30 ml Toluol wurde eine Lösung von 1 g 3-[2,3-Dichlor-4-(chlorformyl-methoxy)benzyl]-2,4-dioxo-1-methyl-6-trifluormethyl-1,2,3,4-tetrahydropyrimidin in 5 ml Toluol getropft. Anschließend rührte man 4 Std. bei ca. 20°C, wonach die Reaktionsmischung mit dreimal 30 ml Wasser gewaschen und dann über Natriumsulfat getrocknet wurde. Schließlich engte man ein. Der Rückstand wurde in 10 ml Diethylether gelöst. Aus der Lösung fällte man das Wertprodukt durch langsame Zugabe von Petrolether. Ausbeute: 0,4 g; Fp.: 151-153°C.

### Vorstufe

### 3-[2,3-Dichlor-4-(chlorformyl-methoxy)benzyl]-2,4-dioxo-1-methyl-6-trifluormethyl-1,2,3,4-tetrahydropyrimidin

Zu einer Lösung von 3-[2,3-Dichlor-4-(hydroxycarbonyl-methoxy)-benzyl]-2,4-dioxo-1-methyl-6-trifluormethyl-1,2,3,4-tetrahydropyrimidin in 50 ml Toluol wurden erst 2 Tropfen Dimethylformamid und dann tropfenweise 0,9 g Thionylchlorid gegeben. Anschließend erhitzte man 4 Std. auf Rückflußtemperatur. Danach wurde die Reaktionsmischung eingeengt. Man erhielt 22 g eines Öls, das ohne Reiniung weiter umgesetzt wurde.

### Beispiel 6

### 3-[2,3-Dichlor-4-(1-[N-(methoxycarbonyl)methyl-N-methyl-aminocarbonyl]ethoxy)benzyl]-2,4-dioxo-1-methyl-6-trifluormethyl-1,2,3,4-tetrahydropyrimidin (nicht mehr erfindungsgemäß)

Zu einer Lösung von 0,35 g Sarcosinmethylesterhydrochlorid in 40 ml Tetrahydrofuran wurden erst 0,69 g Kaliumcarbonat und dann eine Lösung von 1 g 3-[2,3-Dichlor-4-(1-chlorformyl-ethoxy)-benzyl]-2,4-dioxo-1-methyl-6-trifluormethyl-1,2,3,4-tetrahydropyrimidin (hergestellt analog der Vorstufe zu Bsp. 5) in 5 ml Tetrahydrofuran gegeben. Anschließend rührte man 52 Std. bei ca. 20°C, wonach das Lösungsmittel abdestilliert wurde. Den Rückstand löste man in 50 ml Methylenchlorid. Die erhaltene organische Phase wurde zunächst mit Wasser gewaschen, dann über Natriumsulfat getrocknet und schließlich eingeengt. Die Reinigung des erhaltenen Rohprodukts erfolgte mittels Kieselgelchromatographie (Laufmittel: Methylenchlorid). Ausbeute: 0,27 g (Öl).

### Beispiel 7

### 3-(2,3-Dichlor-4-[1-(methoxyaminocarbonyl)ethoxy]benzyl)-2,4-dioxo-1-methyl-6-trifluormethyl-1,2,3,4-tetrahydropyrimidin (nicht mehr erfindungsgemäß)

Zu 2,67 g einer 25 gew.%igen Lösung von Methoxyaminhydrochlorid in 100 ml Toluol wurden bei ca. 20°C 2,2 ml Triethylamin getropft. Anschließend versetzte man die erhaltene Mischung tropfenweise mit einer Lösung von 3,4 g 3-[2,3-Dichlor-4-(1-chlorformylethoxy)benzyl]-2,4-dioxo-1-methyl-6-trifluormethyl-1,2,3,4-tetrahydropyrimidin (hergestellt analog der Vorstufe zu Bsp. 5) in 10 ml Toluol. Nach 5 Std. rühren wurde die organische Phase abgetrennt, mit Wasser gewaschen, dann über Natriumsulfat getrocknet und schließlich eingeengt. Die Reinigung des erhaltenen Öls erfolgte mittels Kieselgelchromatographie (Laufmittel: Methylenchlorid). Ausbeute: 1,1 g; Fp.: 80°C (Zers.).

### Beispiel 8

### 3-(2,3-Dichlor-4-[1-(methoxycarbonylmethoxy)-1-(methoxyimino)-prop-2-yloxy]benzyl)-2,4-dioxo-1-methyl-6-trifluormethyl-1,2,3,4-tetrahydropyrimidin (Verbindung Nr. Ia.48)

Zu einer Lösung von 0,8 g 3-(2,3-Dichlor-4-[1-(methoxyaminocarbonyl)ethoxy]benzyl)-2,4-dioxo-1-methyl-6-trifluormethyl-1,2,3,4-tetrahydropyrimidin in 40 ml Aceton wurden 0,27 g Kaliumcarbonat gegeben. Anschließend versetzte man die erhaltene Mischung tropfenweise mit einer Lösung von 0,20 g Bromessigsäuremethylester in 5 ml Aceton. Dann rührte man 52 Std. bei ca. 20°C, wonach das Lösungsmittel abdestilliert wurde. Den Rückstand nahm man in 50 ml Methylenchlorid auf. Die erhaltene organische Phase wurde zunächst mit zweimal 50 ml Wasser gewaschen, dann über Natriumsulfat getrocknet und schließlich eingeengt. Die Reinigung des erhaltenen Rohprodukts erfolgte mittels Kieselgelchromatographie (Laufmittel: Methylenchlorid). Ausbeute: 0,2 g (Öl).

In der folgenden Tabelle 2 sind die physikalischen Daten weiterer erfindungsgemäßer Verbindungen I, die auf ähnliche Weise hergestellt wurden, aufgeführt:

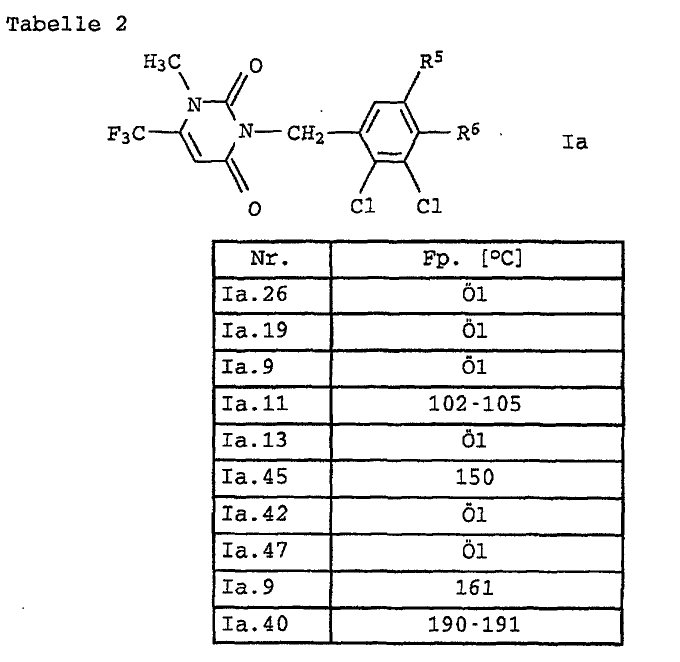

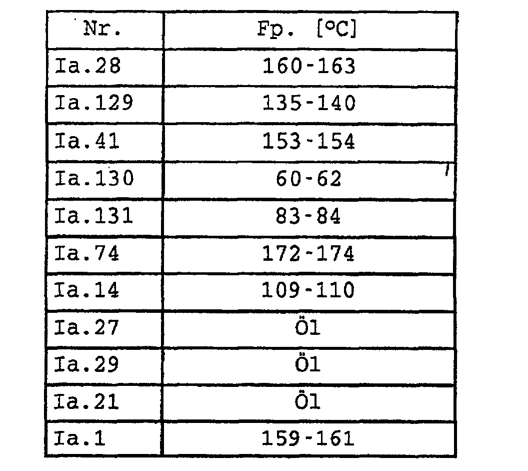

### Anwendungsbeispiele (herbizide Wirksamkeit)

Die herbizide Wirkung der substituierten 1-Methyl-3-benzyluracile I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 7,8 oder 3,9 g/ha a.S. (aktive Substanz).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Triticum aestivum | Winterweizen | winter wheat |
| Abutilon theophrasti | Chinesischer Hanf | velvet leaf |
| Amaranthus retroflexus | Zurückgekrümmter Fuchsschwanz | redroot pigweed |
| Solanum nigrum | Schwarzer Nachtschatten | black nightshade |

Bei Aufwandmengen von 7,8 und 3,9 g/ha a.S. zeigte die Verbindung Nr. Ia.5 im Nachauflaufverfahren eine sehr gute und selektive herbizide Wirkung gegen Abutilon theophrasti, Amaranthus retroflexus und Solanum nigrum in der Kultur Weizen.

### Anwendungsbeispiele (desikkative/defoliante Wirksamkeit)

Als Testpflanzen dienten junge, 4blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnaß mit wäßrigen Aufbereitungen der Wirkstoffe (unter Zusatz von 0,15 Gew.-% des Fettalkoholalkoxylats Plurafac® LF 700¹⁾, bezogen auf die Spritzbrühe) blattbehandelt. Die ausgebrachte Wassermenge betrug umgerechnet 1000 1/ha. Nach 13 Tagen wurde die Anzahl der abgeworfenen Blätter und der Grad der Entblätterung in % bestimmt.
¹⁾ ein schaumarmes, nichtionisches Tensid der BASF AG

Bei den unbehandelten Kontrollpflanzen trat kein Blattfall auf.

## Patentansprüche

1. Substituierte 1-Methyl-3-benzyluracile der Formel I 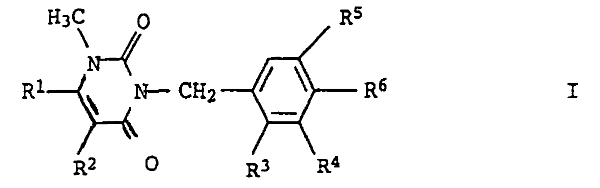 in der die Variablen folgende Bedeutungen haben:
R¹ C₁-C₄-Halogenalkyl;
R² Wasserstoff;
R³ Halogen;
R⁴ Cyano, Halogen oder C₁-C₄-Alkoxy;
R⁵ Wasserstoff;
R⁶ C₁-C₆-Alkoxy oder C₃-C₆-Alkenyloxy, wobei jeder dieser beiden Reste einen oder zwei der folgenden Substituenten tragen kann:
- die Phenylgruppe, die unsubstituiert sein oder ein bis drei Substituenten tragen kann, jeweils ausgewählt aus der Gruppe bestehend aus Halogen, Nitro, Cyano, C₁-C₆-Alkyl, C₁-C₆-Halogenalkyl, C₁-C₆-Alkoxy und (C₁-C₆-Alkoxy)carbonyl,
- eine Gruppe -CO-R⁹, -CO-OR⁹, -CO-SR⁹ oder -CO-N(R⁹)R¹⁰,
- eine Gruppe =N-OR²⁰ oder -C(R²¹)=N-OR²⁰;
- CH(NR²⁷), -CH=C(R¹⁴)-CO-R¹⁵,
-CH=C(R¹⁴)-CO-N (R¹⁹)-OR²⁰, -CH=C(R¹⁴)-C(R²¹)=N-OR²⁰,
-N(R²³)R²⁴, -CO-N(R¹⁹)-OR²⁰, -C(R²¹)=N-OR²⁰, -COO-R²² oder
-CON(R²³)R²⁴;
R⁹ C₁-C₃-Alkyl, C₃-C₄-Alkenyl oder (C₁-C₃-Alkoxy)carbonyl-C₁-C₃-alkyl;
R¹⁰ Wasserstoff, C₁-C₃-Alkoxy, (C₁-C₃-Alkoxy)carbonyl-C₁-C₃-alkoxy oder C₃-C₄-Alkenyloxy;
R¹⁴ Wasserstoff, Halogen oder C₁-C₆-Alkyl;
R¹⁵ O-R²² oder -N(R²³)R²⁴;
R¹⁹ Wasserstoff, C₁-C₃-Alkyl oder C₃-C₄-Alkenyl, wobei die beiden letzten Gruppen jeweils einen der folgenden Reste tragen können: Chlor, (C₁-C₆-Alkyl)carbonyl, (C₁-C₆-Alkoxy)carbonyl oder (C₃-C₆-Alkenyloxy)carbonyl; oder (C₁-C₆-Alkylamino)carbonyl oder Di-(C₁-C₆-alkyl)aminocarbonyl;
R²⁰ C₁-C₄-Alkyl, C₃-C₄-Alkenyl oder (C₁-C₃-Alkoxy)carbonyl-C₁-C₃-alkyl;
R²¹ C₃-C₄-Alkinyloxy, C₁-C₃-Alkoxy oder C₃-C₆-Alkenyloxy, wobei die beiden letzten Reste jeweils einen der folgenden Substituenten tragen können: (C₁-C₃-Alkoxy)carbonyl, (C₁-C₃-Alkylamino)carbonyl oder Dimethylaminocarbonyl;
R²² eine der Bedeutungen von R¹⁹;
R²³, R²⁴ unabhängig voneinander Wasserstoff, C₁-C₃-Alkyl, (C₁-C₃-Alkyl)carbonyl, C₁-C₃-Alkylsulfonyl oder Phenylsulfonyl, dessen Phenylring einen Halogen-, C₁-C₃-Alkyloder C₁-C₃-Alkoxy-Substituenten trägt;
R²⁷ C₁-C₆-Alkoxy, C₃-C₆-Alkenyloxy, (C₁-C₆-Alkoxy)carbonyl-C₁-C₆-alkoxy oder Benzyloxy;
sowie die landwirtschaftlich brauchbaren Salze und Enolether der Verbindungen I.

2. Verwendung der substituierten l-Methyl-3-benzyluracile der Formel I und ihrer landwirtschaftlich brauchbaren Salze und Enolether, gemäß Anspruch 1, als Herbizide oder zur Desikkation/Defoliation von Pflanzen.

3. Herbizides Mittel, enthaltend eine herbizid wirksame Menge mindestens eines substituierten 1-Methyl-3-benzyluracils der Formel I oder eines landwirtschaftlich brauchbaren Salzes oder Enolethers von I, gemäß Anspruch 1, und mindestens einen inerten flüssigen und/oder festen Trägerstoff sowie gewünschtenfalls mindestens einen oberflächenaktiven Stoff.

4. Mittel zur Desikkation und/oder Defoliation von Pflanzen, enthaltend eine desikkant und/oder defoliant wirksame Menge mindestens eines substituierten 1-Methyl-3-benzyluracils der Formel I oder eines landwirtschaftlich brauchbaren Salzes oder Enolethers von I, gemäß Anspruch 1, und mindestens einen inerten flüssigen und/oder festen Trägerstoff sowie gewünschtenfalls mindestens einen oberflächenaktiven Stoff.

5. Verfahren zur Herstellung von herbizid wirksamen Mitteln, **dadurch gekennzeichnet, daß** man eine herbizid wirksame Menge mindestens eines substituierten 1-Methyl-3-benzyluracils der Formel I oder eines landwirtschaftlich brauchbaren Salzes oder Enolethers von I, gemäß Anspruch 1, und mindestens einen inerten flüssigen und/oder festen Trägerstoff sowie gewünschtenfalls mindestens einen oberflächenaktiven Stoff mischt.

6. Verfahren zur Herstellung von desikkant und/oder defoliant wirksamen Mitteln, **dadurch gekennzeichnet, daß** man eine desikkant und/oder defoliant wirksame Menge mindestens eines substituierten 1-Methyl-3-benzyluracils der Formel I oder eines landwirtschaftlich brauchbaren Salzes oder Enolethers von I, gemäß Anspruch 1, und mindestens einen inerten flüssigen und/oder festen Trägerstoff sowie gewünschtenfalls mindestens einen oberflächenaktiven Stoff mischt.

7. Verfahren zur Bekämpfung von unerwünschtem Pflanzenwuchs, **dadurch gekennzeichnet, daß** man eine herbizid wirksame Menge mindestens eines substituierten 1-Methyl-3-benzyluracils der Formel I oder eines landwirtschaftlich brauchbaren Salzes oder Enolethers von I, gemäß Anspruch 1, auf Pflanzen, deren Lebensraum oder auf Saatgut einwirken läßt.

8. Verfahren zur Desikkation und/oder Defoliation von Pflanzen, **dadurch gekennzeichnet, daß** man eine desikkant und/oder defoliant wirksame Menge mindestens eines substituierten 1-Methyl-3-benzyluracils der Formel I oder eines landwirtschaftlich brauchbaren Salzes von I, gemäß Anspruch 1, auf Pflanzen einwirken läßt.

9. Verfahren nach Anspruch 8, **dadurch gekennzeichnet, daß** man Baumwolle behandelt.

10. Verfahren zur Herstellung von substituierten 1-Methyl-3-benzyluracilen der Formel I gemäß Anspruch 1, **dadurch gekennzeichnet, daß** man einen Enaminester der Formel III 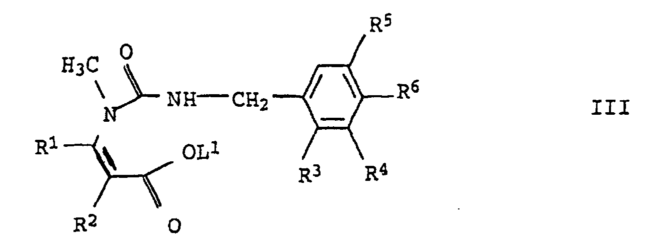 oder ein Enamincarboxylat der Formel IV 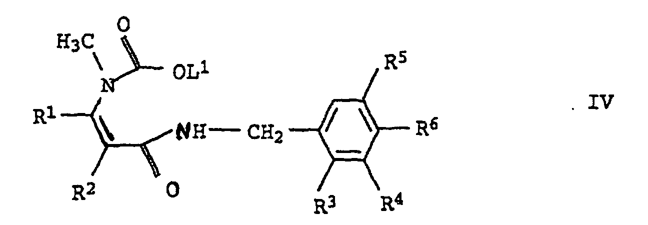 wobei L¹ jeweils für niedermolekulares Alkyl oder Phenyl steht und die Substituenten R¹ bis R⁶ die in Anspruch 1 angegebenen Bedeutungen haben, in Gegenwart einer Base cyclisiert.

11. Verfahren zur Herstellung von substituierten 1-Methyl-3-benzyluracilen der Formel I gemäß Anspruch 1, **dadurch gekennzeichnet, daß** man ein 1-H-3-Benzyluracil der Formel V 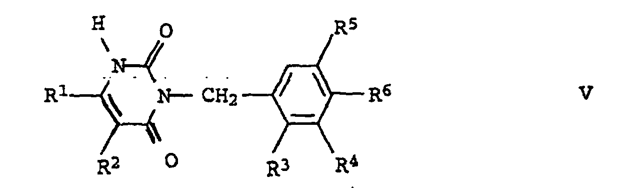 in der die Substituenten R¹ bis R⁶ die in Anspruch 1 angegebenen Bedeutungen haben, methyliert.

12. Verfahren zur Herstellung von substituierten 1-Methyl-3-benzyluracilen der Formel I gemäß Anspruch 1, **dadurch gekennzeichnet, daß** man ein 1-Methyl-6-halogenalkyluracil der Formel VIII 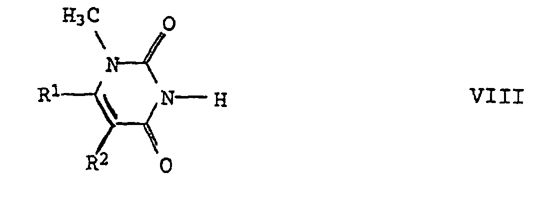 in Gegenwart einer Base, oder ein Alkalimetallsalz von VIII, mit einem Benzylhalogenid der Formel IX 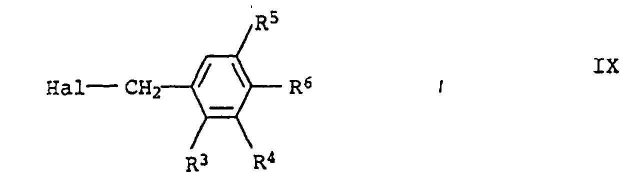 wobei Hal für Halogen steht und die Substituenten R³ bis R⁶ die in Anspruch 1 angegebenen Bedeutungen haben, umsetzt.

13. Enaminester der Formel III 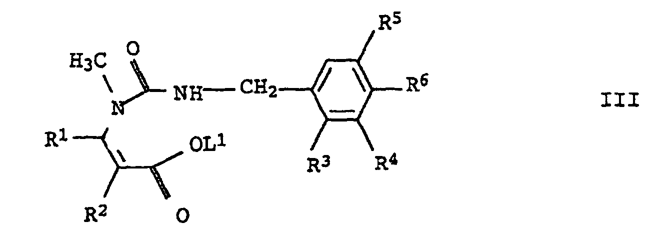 in der L¹ für C₁-C₆-Alkyl oder Phenyl steht und die Substituenten R¹ bis R⁶ die in Anspruch 1 angegebenen Bedeutungen haben.

14. Enamincarboxylate der Formel IV 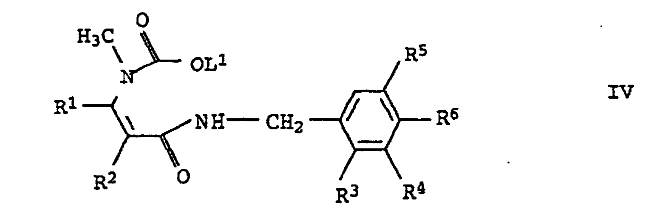 in der L¹ für C₁-C₆-Alkyl oder Phenyl steht und die Substituenten R¹ bis R⁶ die in Anspruch 1 angegebenen Bedeutungen haben.

15. 1-Methyl-6-halogenalkyluracile der Formel VIII 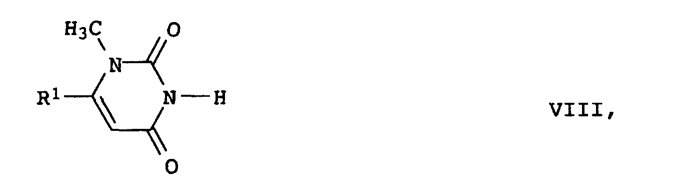 wobei R¹ für C₁-C₄-Halogenalkyl steht.

16. Eine Verbindung der Formel VIII nach Anspruch 15, in der R¹ für Trifluormethyl steht.

## Claims

1. A substituted 1-methyl-3-benzyluracil of the formula I 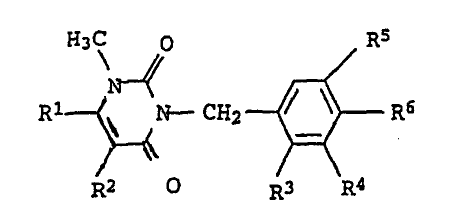 where:
R¹ is C₁-C₄-haloalkyl;
R² is hydrogen;
R³ is halogen;
R⁴ is cyano, halogen or C₁-C₄-alkoxy;
R⁵ is hydrogen;
R⁶ is C₁-C₆-alkoxy or C₃-C₆-alkenyloxy, where each of these two radicals may carry one or two of the following substituents:
- phenyl which may be unsubstituted or may carry up to three substituents, in each case selected from the group consisting of halogen, nitro, cyano, C₁-C₆-alkyl, C₁-C₆-haloalkyl, C₁-C₆-alkoxy and (C₁-C₆-alkoxy)carbonyl,
- a group -CO-R⁹, -CO-OR⁹, -CO-SR⁹ or -CO-N(R⁹)R¹⁰,
- a group =N-OR²⁰ or -C(R²¹)=N-OR²⁰;
- CH(NR²⁷), -CH=C(R¹⁴)-CO-R¹⁵, -CH=C(R¹⁴)-CO-N(R¹⁹)-OR²⁰,
-CH=C(R¹⁴)-C(R²¹)-N-OR²⁰, -N(R²³)R²⁴, -CO-N(R¹⁹)-OR²⁰,
-C(R²¹)=N-OR²⁰, -COO-R²² or -CON(R²³)R²⁴;
R⁹ is C₁-C₃-alkyl, C₃-C₄-alkenyl or (C₁-C₃-alkoxy)-carbonyl-C₁-C₃-alkyl;
R¹⁰ is hydrogen, C₁-C₃-alkoxy, (C₁-C₃-alkoxy)carbonyl-C₁-C₃-alkoxy or C₃- or C₄-alkenyloxy;
R¹⁴ is hydrogen, halogen or C₁-C₆-alkyl;
R¹⁵ is O-R²² or -N(R²³)R²⁴;
R¹⁹ is hydrogen, C₁-C₃-alkyl or C₃- or C₄-alkenyl, where the last two groups may carry in each case one of the following radicals: chlorine, (C₁-C₆-alkyl)carbonyl, (C₁-C₆-alkoxy)carbonyl or (C₃-C₆-alkenyloxy)carbonyl; or (C₁-C₆-alkylamino)carbonyl or di(C₁-C₆-alkyl)-aminocarbonyl;
R²⁰ is C₁-C₄-alkyl, C₃-C₄-alkenyl or (C₁-C₃-alkoxy)carbonyl-C₁-C₃-alkyl;
R²¹ is C₃-C₄-alkynyloxy, C₁-C₃-alkoxy or C₃-C₆-alkenyloxy, where the last two radicals may carry in each case one of the following substituents: (C₁-C₃-alkoxy)carbonyl, (C₁-C₃-alkylamino)carbonyl or dimethylaminocarbonyl;
R²² has one of the meanings of R¹⁹;
R²³ and R²⁴, independently of one another, are hydrogen, C₁-C₃-alkyl, (C₁-C₃-alkyl)carbonyl, C₁-C₃-alkylsulfonyl or phenylsulfonyl whose phenyl ring carries a halogen, C₁-C₃-alkyl or C₁-C₃-alkoxy substituent;
R²⁷ is C₁-C₆-alkoxy, C₃-C₆-alkenyloxy, (C₁-C₆-alkoxy)carbonyl-C₁-C₆-alkoxy or benzyloxy;
and the agriculturally useful salts and enol ethers of the compounds I.

2. Use of a substituted 1-methyl-3-benzyluracil of the formula I or of agriculturally usefyl of salts or enol ethers thereof, as claimed in claim 1, as herbicides or for desiccating/defoliating plants.

3. Herbicide containing a herbicidal amount of at least one substituted 1-methyl-3-benzyluracil of the formula I or of an agriculturally useful salt or enol ether of I, as claimed in claim 1, and at least one inert liquid or solid carrier and, if desired, at least one surfactant.

4. A plant desiccant or defoliant, containing an amount, having a desiccant or defoliant action, of at least one substituted 1-methyl-3-benzyluracil of the formula I or of an agriculturally useful salt or enol ether of I, as claimed in claim 1, and at least one inert liquid or solid carrier and, if desired, at least one surfactant.

5. A process for the preparation of a herbicide, wherein a herbicidal amount of at least one substituted 1-methyl-3-benzyluracil of the formula I or of an agriculturally useful salt or enol ether of I, as claimed in claim 1, and at least one inert liquid or solid carrier and, if desired, at least one surfactant are mixed.

6. A process for the preparation of a desiccant or defoliant, wherein an amount, having a desiccant or defoliant action, of at least one substituted 1-methyl-3-benzyluracil of the formula I or of an agriculturally useful salt or enol ether of I, as claimed in claim 1, and at least one inert liquid or solid carrier and, if desired, at least one surfactant are mixed.

7. A method for controlling undesirable plant growth, wherein a herbicidal amount of at least one substituted 1-methyl-3-benzyluracil of the formula I or of an agriculturally useful salt or enol ether of I as claimed in claim 1, is allowed to act on plants or their habitat or on seed.

8. A method for desiccating or defoliating plants, wherein an amount, having a desiccant or defoliant action, of at least one substituted 1-methyl-3-benzyluracil of the formula I or of an agriculturally useful salt of I, as claimed in claim 1, is allowed to act on plants.

9. A method as claimed in claim 8, wherein cotton is treated.

10. A process for the preparation of a substituted 1-methyl-3-benzyluracil of the formula I as claimed in claim 1, wherein an enaminoester of the formula III 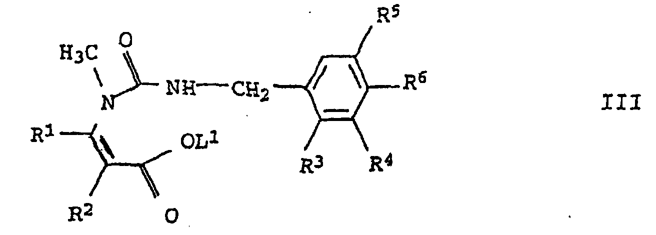 or an enaminocarboxylate of the formula IV 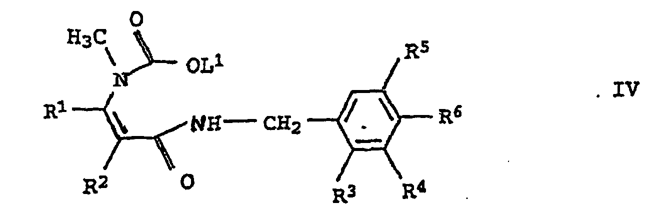 where L¹ in each case is low molecular weight alkyl or phenyl and R¹ to R⁶ each have the meanings stated in claim 1, is cyclized in the presence of a base.

11. A process for the preparation of a substituted 1-methyl-3-benzyluracil of the formula I as claimed in claim 1, wherein a 1-H-3-benzyluracil of the formula V 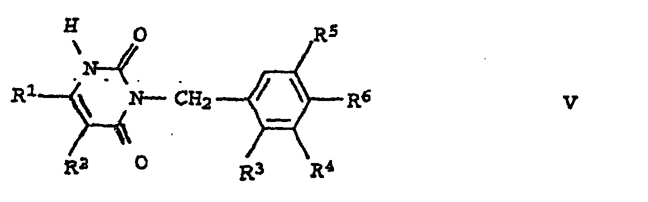 where R¹ to R⁶ have the meanings stated in claim 1, is methylated.

12. A process for the preparation of a substituted 1-methyl-3-benzyluracil of the formula I as claimed in claim 1, wherein a 1-methyl-6-haloalkyluracil of the formula VIII 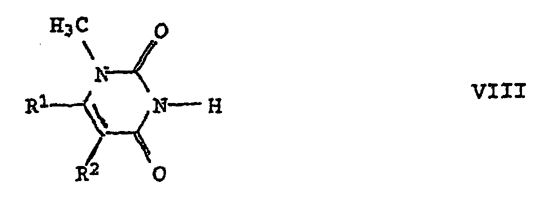 in the presence of a base, or an alkali metal salt of VIII, is reacted with a benzyl halide of the formula IX 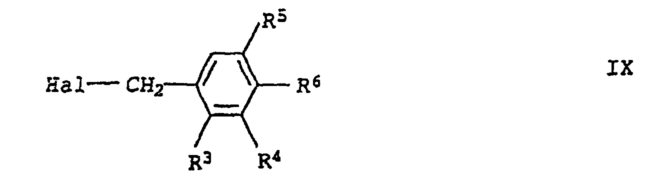 where Hal is halogen and R³ to R⁶ each have the meanings stated in claim 1.

13. An enaminoester of the formula III 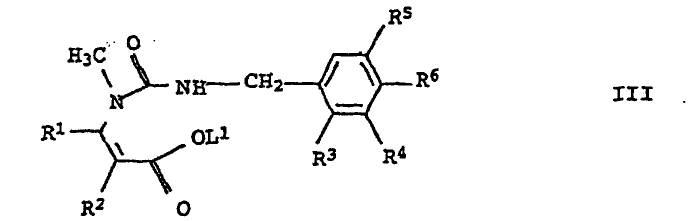 where L¹ is C₁-C₆-alkyl or phenyl and R¹ to R⁶ each have the meaning stated in claim 1.

14. An enaminocarboxylate of the formula IV 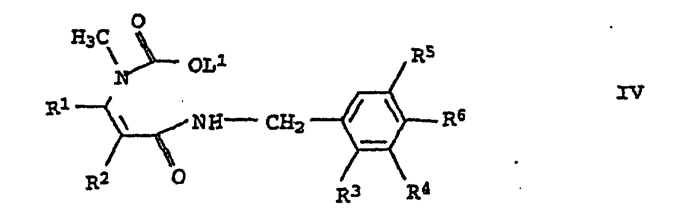 where L¹ is C₁-C₆-alkyl or phenyl and R¹ to R⁶ each have the meanings stated in claim 1.

15. A 1-methyl-6-haloalkyluracil of the formula VIII 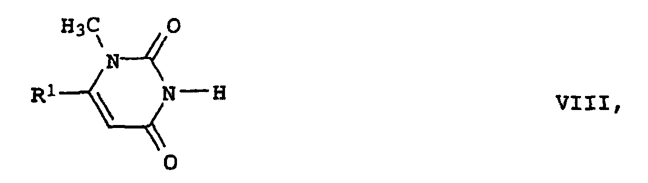 where R¹ is C₁-C₄-haloalkyl.

16. A compound of the formula VIII as claimed in claim 15, where R¹ is trifluoromethyl.

## Revendications

1. 1-méthyl-3-benzyluraciles substitués de formule I 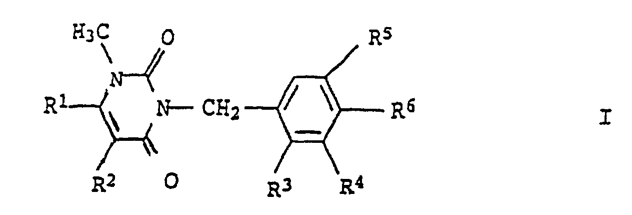 dans laquelle les symboles ont les significations suivantes :
R¹ : un groupe halogénoalkyle en C1-C4 ;
R² : l'hydrogène;
R³ : un halogène ;
R⁴ : un groupe cyano, un halogène ou un groupe alcoxy en C1-C4 ;
R⁵ : l'hydrogène;
R⁶ : un groupe alcoxy en C1-C6 ou alcényloxy en C3-C6, chacun de ces deux groupes pouvant porter un ou deux des substituants suivants :
- le groupe phényle, non substitué ou portant un à trois substituants choisis parmi les halogènes, les groupes nitro, cyano, alkyle en C1-C6, halogénoalkyle en C1-C6, alcoxy en C1-C6 et (alcoxy en C1-C6)carbonyle,
- un groupe -CO-R⁹, -CO-OR⁹, -CO-SR⁹ ou -CO-N(R⁹)R¹⁰,
- un groupe =N-OR²⁰ ou -C(R²¹)=N-OR²⁰;
- un groupe -CH(NR²⁷), -CH=C(R¹⁴)-CO-R¹⁵,
- CH=C(R¹⁴)-CO-N(R¹⁹)-OR²⁰, -CH=C(R¹⁴)-C(R²¹)=N-OR²⁰,
-N(R²³)R²⁴, -CO-N(R¹⁹)-OR²⁰, -C(R²¹)=N-OR²⁰, -COO-R²² ou
-CON(R²³)R²⁴;
R⁹ : un groupe alkyle en C1-C3, alcényle en C3-C4 ou (alcoxy en C1-C3)carbonyl-alkyle en C1-C3 ;
R¹⁰: l'hydrogène, un groupe alcoxy en C1-C3, (alcoxy en C1-C3)carbonyl-alcoxy en C1-C3 ou alcényloxy en C3-C4 ;
R¹⁴ : l'hydrogène, un halogène ou un groupe alkyle en C1-C6 ;
R¹⁵ : un groupe O-R²² ou -N(R²³)R²⁴;
R¹⁹: l'hydrogène, un groupe alkyle en C1-C3 ou alcényle en C3-C4, ces deux derniers groupes pouvant porter chacun l'un des substituants suivants : le chlore, un groupe (alkyle en C1-C6)carbonyle, (alcoxy en C1-C6)carbonyle ou (alcényloxy en C3-C6)carbonyle ;
ou un groupe (alkylamino en C1-C6)carbonyle ou di(alkyle en C1-C6)aminocarbonyle ;
R²⁰ : un groupe alkyle en C1-C4, alcényle en C3-C4 ou (alcoxy en C1-C3)carbonylalkyle en C1-C3 ;
R²¹ : un groupe alcynyloxy en C3-C4, alcoxy en C1-C3 ou alcényloxy en C3-C6, ces deux derniers groupes pouvant porter chacun l'un des substituants suivants : (alcoxy en C1-C3)carbonyle, (alkylamino en C1-C3)carbonyle ou diméthylaminocarbonyle ;
R²² a l'une des significations de R¹⁹ ;
R²³, R²⁴ : indépendamment l'un de l'autre : l'hydrogène, un groupe alkyle en C1-C3, (alkyle en Cl-C3)carbonyle, alkylsulfonyle en C1-C3 ou phénylsulfonyle dont le cycle phényle porte un substituant halogéno, alkyle en C1-C3 ou alcoxy en C1-C3 ;
R²⁷ : un groupe alcoxy en C1-C6, alcényloxy en C3-C6, (alcoxy en C1-C6)carbonyl-alcoxy en C1-C6 ou benzyloxy ;
et les sels et éthers d'énols, aptes aux applications agricoles, des composés I.

2. Utilisation des 1-méthyl-3-benzyluraciles substitués de formule I et de leurs sels et éthers d'énols aptes aux applications agricoles selon revendication 1 en tant qu'herbicides ou pour la dessiccation/défoliation de végétaux.

3. Produit herbicide contenant une quantité herbicide efficace d'au moins un 1-méthyl-3-benzyluracile substitué de formule I ou de l'un de ses sels ou éthers d'énols aptes aux applications agricoles selon revendication 1 et au moins un véhicule liquide et/ou solide inerte ainsi que, lorsqu'on le désire, au moins une substance tensio-active.

4. Produit pour la dessiccation et/ou la défoliation de végétaux, contenant une quantité dessiccante et/ou défoliante efficace d'au moins un 1-méthyl-3-benzyluracile substitué de formule I ou d'un de ses sels ou éthers d'énols aptes aux applications agricoles selon la revendication 1 et au moins un véhicule liquide et/ou solide inerte ainsi que, si on le désire, au moins une substance tensio-active.

5. Procédé pour la préparation de produits à activité herbicide **caractérisé par le fait que** l'on mélange une quantité herbicide efficace d'au moins un 1-méthyl-3-benzyluracile substitué de formule I ou de l'un de ses sels ou éthers d'énols aptes aux applications agricoles selon la revendication 1 et au moins un ^{v}éhicule liquide et/ou solide inerte ainsi que, si on le désire, au moins une substance tensio-active.

6. Procédé pour la préparation de produits à activité dessiccante et/ou défoliante **caractérisé par le fait que** l'on mélange une quantité dessiccante et/ou défoliante efficace d'au moins un 1-méthyl-3-benzyluracile substitué de formule I ou d'un sel ou éther d'énol apte aux applications agricoles de I selon la revendication 1 et au moins un véhicule liquide et/ou solide inerte ainsi que, si on le désire, au moins une substance tensio-active.

7. Procédé pour combattre les croissances de végétaux indésirables, **caractérisé en ce que** l'on fait agir une quantité herbicide efficace d'au moins un 1-méthyl-3-benzyluracile substitué de formule I ou de l'un de ses sels ou éthers d'énols aptes aux applications agricoles selon la revendication 1 sur les végétaux, leur habitat ou sur les semences.

8. Procédé pour la dessiccation et/ou la défoliation de végétaux, **caractérisé par le fait que** l'on fait agir une quantité dessiccante et/ou défoliante efficace d'au moins un 1-méthyl-3-benzyluracile substitué de formule I ou de l'un de ses sels aptes aux applications agricoles selon la revendication 1, sur les végétaux.

9. Procédé selon revendication 8, **caractérisé par le fait que** l'on traite le coton.

10. Procédé pour la préparation des 1-méthyl-3-benzyluraciles substitués de formule I de la revendication 1, **caractérisé par le fait que** l'on cyclise en présence d'une base un énamine-ester de formule III 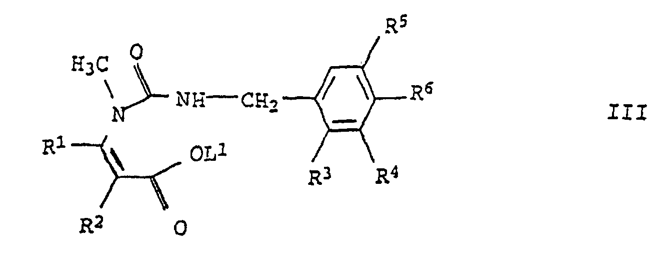 ou un énamine-carboxylate de formule IV 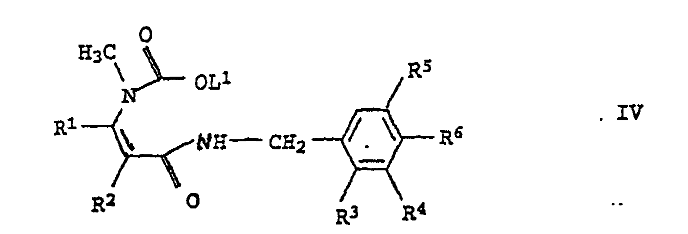 L¹ représentant dans chaque cas un groupe alkyle inférieur ou phényle et les symboles R¹ à R⁶ ayant les significations indiquées dans la revendication 1.

11. Procédé pour la préparation des 1-méthyl-3-benzyluraciles substitués de formule I de la revendication 1, **caractérisé par le fait que** l'on méthyle un 1-H-3-benzyluracile de formule V 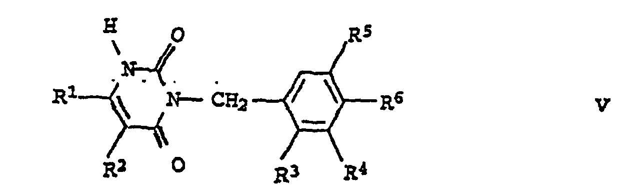 dans laquelle les symboles R¹ à R⁶ ont les significations indiquées dans la revendication 1.

12. Procédé pour la préparation des 1-méthyl-3-benzyluraciles substitués de formule I de la revendication 1, **caractérisé par le fait que** l'on fait réagir un 1-méthyl-6-halogénoalkyluracile de formule VIII 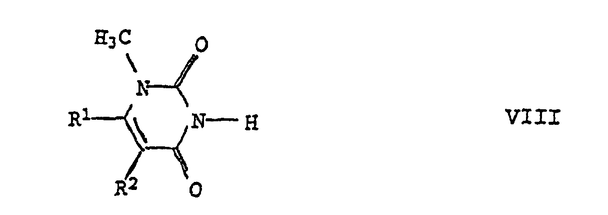 en présence d'une base, ou bien un sel de métal alcalin de VIII, avec un halogénure de benzyle de formule IX 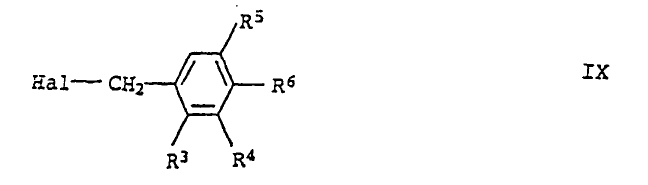 dans laquelle Hal représente un halogène et R³ à R⁶ ont les significations indiquées dans la revendication 1.

13. Enamine-esters de formule III 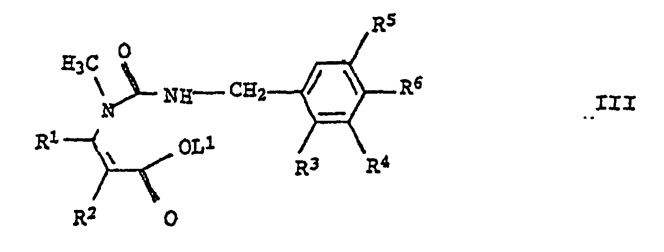 dans laquelle L¹ représente un groupe alkyle en C1-C6 ou phényle et les symboles R¹ à R⁶ ont les significations indiquées dans la revendication 1.

14. Enamine-carboxylates de formule IV 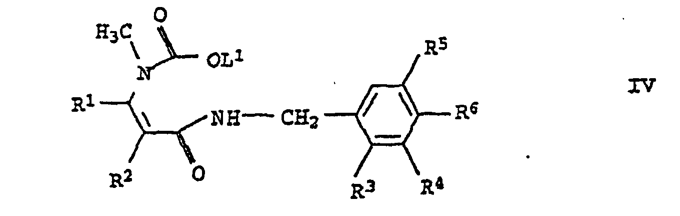 dans laquelle L¹ représente un groupe alkyle en C1-C6 ou phényle et les symboles R¹ à R⁶ ont les significations indiquées dans la revendication 1.

15. 1-méthyl-6-halogénoalkyluraciles de formule VIII 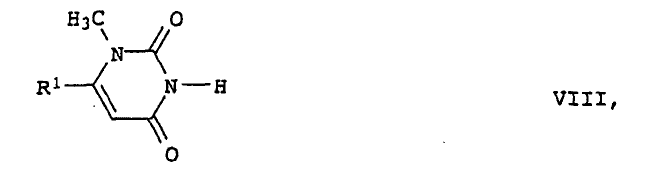 dans laquelle R¹ représente un groupe halogénoalkyle en C1-C4.

16. Un composé de formule VIII de la revendication 15, dans laquelle R¹ représente un groupe trifluorométhyle.
